(19) Europäisches Patentamt
European Patent Office
Office européen des brevets

(11) Veröffentlichungsnummer: **0 126 709 B1**

# (12) EUROPÄISCHE PATENTSCHRIFT

(45) Veröffentlichungstag der Patentschrift: **03.04.91**

(51) Int. Cl.5: **C07D 205/08**

(21) Anmeldenummer: **84810136.6**

(22) Anmeldetag: **22.03.84**

Die Akte enthält technische Angaben, die nach dem Eingang der Anmeldung eingereicht wurden und die nicht in dieser Patentschrift enthalten sind.

## (54) Verfahren zur Herstellung von optisch aktiven Azetidinonen.

(30) Priorität: **28.03.83 CH 1678/83**

(43) Veröffentlichungstag der Anmeldung:
**28.11.84 Patentblatt 84/48**

(45) Bekanntmachung des Hinweises auf die Patenterteilung:
**03.04.91 Patentblatt 91/14**

(84) Benannte Vertragsstaaten:
**BE CH DE FR GB IT LI NL SE**

(56) Entgegenhaltungen:
**EP-A- 0 023 887**
**EP-A- 0 041 768**

**TETRAHEDRON LETTERS, Band 24, Nr. 10, 1983, Oxford, GB; H. YANAGISAWA et al. "Synthesis of optically active azetidin-2-ones from 1-threonine", Seiten 1037-1040**

**TETRAHEDRON LETTERS, Band 22, Nr. 51, 1981, Oxford, GB; M. SHIOZAKI et al. "Stereocontrolled syntheses of chiral intermediates of thienamycin from threonines", Seiten 5205-5208**

**Chemical Abstracts Band 98, Nr. 15, 11. April 1983, Columbus, Ohio, USA; Seite 609, Spalte 2, Abstract Nr. 125761d & JP-A-57-163362**

(73) Patentinhaber: **CIBA-GEIGY AG**
**Klybeckstrasse 141**
**CH-4002 Basel(CH)**

(72) Erfinder: **Ernest, Ivan, Dr.**
**Rüttihard 10**
**CH-4127 Birsfelden(CH)**
Erfinder: **Kalvoda, Jaroslav, Dr.**
**Leimgrubenweg 21**
**CH-4102 Binningen(CH)**
Erfinder: **Fröstl, Wolfgang, Dr.**
**St. Johanns-Vorstadt 6/2**
**CH-4056 Basel(CH)**

**EP 0 126 709 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft ein verbessertes Verfahren zur Herstellung von (3S)-3,4-trans-disubstituierten Azetidinonen der Formel

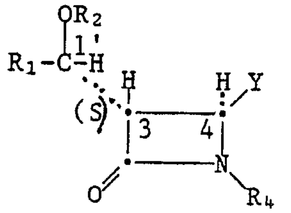

(I) ,

worin $R_1$ Wasserstoff oder $C_1$-$C_7$-Alkyl, $R_2$ Wasserstoff oder eine Hydroxyschutzgruppe, Y die Gruppen -S(=O)-$R_3$ oder -S(=O)$_2$-$R_3$, worin $R_3$ einen tertiären $C_4$-$C_7$-Alkyl-, Cycloalkyl-$C_1$-$C_7$-Alkyl-, Aryl-, oder einen durch $C_1$-$C_7$-Alkoxy, $C_1$-$C_7$-Alkyl, Nitro und/oder Halogen substituierten Arylrest wie 4-Methoxyphenyl, 4-Nitrophenyl, 2-, 3-, oder 4-Chlorphenyl, Tolyl, oder einen Triaryl-$C_1$-$C_7$-Alkylrest, welcher jeweils durch das tertiäre C-Atom mit dem Schwefelatom verbunden ist, darstellt, oder die Gruppe -C(=O)-O-$R_3'$ , worin $R_3'$ die gleiche Bedeutung besitzt wie $R_3$ und $R_3'$ durch jeweils das tertiäre C-Atom mit dem Sauerstoffatom der Carboxygruppe verbunden ist, oder eine Carboxyschutzgruppe darstellt, und $R_4$ Wasserstoff oder eine Aminoschutzgruppe $R_4'$ bedeutet, und worin das 4-C-Atom des Azetidinonrings die R-Konfiguration hat, wenn Y die Gruppen -S(=O)-$R_3$ oder -S(=O)$_2$-$R_3$ bedeutet, und die S-Konfiguration hat, wenn Y die Gruppe -C(=O)-O-$R_3'$ bedeutet, und wobei das 1'-C-Atom der Seitenkette die R- oder die S-Konfiguration hat, wenn $R_1$ $C_1$-$C_7$-Alkyl bedeutet, durch Ringschluss einer Verbindung der Formel (II)

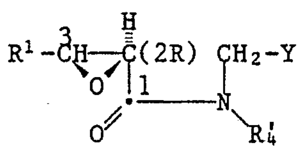

(II)

worin Y und $R_1$ die unter Formel (I) genannten Bedeutungen haben, $R_4'$ eine geeignete Aminoschutzgruppe darstellt und das 2-C-Atom die R- und, wenn $R_1$ $C_1$-$C_7$-Alkyl bedeutet, das 3-C-Atom die R- oder die S-Konfiguration hat, oder einer Verbindung der Formel (III)

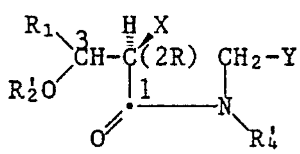

(III)

worin Y und $R_1$ die unter Formel (I) genannten Bedeutungen haben, $R_2'$ Wasserstoff oder eine unter den Bedingungen des Ringschlussverfahrens nicht abspaltbare Hydroxyschutzgruppe, $R_4'$ eine geeignete Aminoschutzgruppe, X eine nukleofuge Abgangsgruppe darstellt und das 2-C-Atom die R- und, wenn $R_1$ $C_1$-$C_7$-Alkyl bedeutet, das 3-C-Atom die R- oder die S-Konfiguration hat, in einem aprotischen, organischen Lösungsmittel unter Ausschluss von Wasser, dadurch gekennzeichnet , dass man den Ringschluss mittels eines Tetra-$C_1$-$C_7$-Alkylammoniumfluorids bei Temperaturen zwischen -10 °C und Raumtemperatur durchführt und, wenn erwünscht, eine erhältliche Verbindung der Formel (I) in eine andere definitionsgemässe Verbindung der Formel (I) umwandelt.

Verbindungen der Formel (I), worin $R_1$ Methyl, $R_2$ Wasserstoff oder Methoxymethyl, Y Benzolsulfonyl und $R_4$ Wasserstoff, 4-Methoxyphenyl oder 3,4-Dimethoxyphenyl bedeutet, und ihre Herstellung durch Ringschluss mit n-Butyllithium bei -50 °C sind durch Yanagisawa H. et al., Tetrahedron Letters 24 [10], 1037-1040 (1983) bekannt geworden.

Verbindungen der Formel I, worin $R_1$ Methyl, $R_2$ Wasserstoff oder Dimethyl-tert-butylsilyl, Y tert-Butoxycarbonyl und $R_4$ 2,4-Dimethoxybenzyl bedeutet, und ihre Herstellung durch Ringschluss mit Lithium-bis-trimethylsilylamin sind durch Shiozaki M. et al., Tetrahedron Letters 22 [51], 5205-5208 (1981) bekannt

geworden.

Aus C.A. 98 (1983), 12 576 1d ist ein analoges Verfahren zur Herstellung von Azetidinonen bekannt das mit n-Butyllithium arbeitet.

Das erfindungsgemässe Verfahren hat den Vorteil, dass Verbindungen der Formel (I) in mit gegenüber dem Stand der Technik vergleichbaren Ausbeuten unter Verwendung eines Cyclisierungsmittels, das besonders günstige Reaktionsbedingungen erlaubt und zudem regenerierbar ist, hergestellt werden.

In der Beschreibung der vorliegenden Erfindung bedeutet der im Zusammenhang mit Gruppen oder Resten, z.B. Niederalkyl, Niederalkoxy, Niederalkanoyl etc., verwendete Ausdruck "Nieder", dass die so bezeichneten Gruppen oder Reste, falls nicht ausdrücklich anders definiert, bis einschliesslich 7, bevorzugt bis einschliesslich 4, C-Atome enthalten.

Die weiter vorn und im folgenden verwendeten allgemeinen Definitionen haben im Rahmen der vorliegenden Beschreibung vorzugsweise die folgenden Bedeutungen:

Niederalkyl $R_1$ ist vorzugsweise Methyl, ferner Ethyl, n-Propyl, Isopropyl, n-Butyl, Isobutyl oder tert-Butyl.

$R_1$ ist vorzugsweise Wasserstoff oder Methyl.

Hydroxyschutzgruppen $R_2$, deren Einführung und Abspaltung sind beispielsweise in "Protective Groups in Organic Chemistry", Plenum Press, London und New York 1973, sowie in "Protective Groups in Organic Chemistry", Wiley, New York 1974 beschrieben.

Eine Hydroxyschutzgruppe $R_2$, ist beispielsweise die Acylgruppe einer substituierten Carbon- oder Sulfonsäure, z.B. durch Halogen, z.B. Fluor oder Chlor, substituiertes Niederalkanoyl, z.B. 2,2-Dichlor- oder 2,2,2-Trifluoracetyl, die z.B. durch Halogen, z.B. Chlor, Phenyl oder p-Nitrophenyl, substituierte Acylgruppe eines Kohlensäurehalbesters, z.B. 2,2,2-Trichlorethoxycarbonyl, Allyloxycarbonyl, Benzyloxycarbonyl oder p-Nitrobenzyloxycarbonyl, 2-Oxa- oder 2-Thiacycloalkyl mit 5 bis 7 Ringatomen, z.B. 2-Tetrahydrofuryl oder 2-Tetrahydropyranyl oder ein Thiaanaloges davon, oder 1-Niederalkoxyniederalkyl, z.B. Methoxymethyl oder 1-Ethoxyethyl.

Eine geeignete Hydroxyschutzgruppe $R_2$ ist vorzugsweise die substituierte Acylgruppe eines Kohlensäurehalbesters, z.B. 2,2,2-Trichlorethoxycarbonyl, Allyloxycarbonyl, Benzyloxycarbonyl oder p-Nitrobenzyloxycarbonyl oder eine Silylgruppe, z.B. Triniederalkylsilyl, z.B. Trimethyl- oder Triethylsilyl, Diniederalkyl-dreifach substituiertes Methylsilyl, z.B. Dimethyl- oder Diethyl-2-ethyl-2-propylsilyl oder Dimethyl-tert-butylsilyl, Diarylniederalkylsilyl, z.B. Diphenyl-tert-butylsilyl, Arylniederalkylsilyl, z.B. tert-Butylmethyl-phenylsilyl, sowie Tri-(niederalkyl)-aryloxydiniederalkylsilyl, z.B. 2,4,6-Tri-(tert-butyl)phenoxydimethylsilyl.

tert-$C_4$-$C_7$-Alkyl $R_3$ ist beispielsweise tert-Butyl oder tert-Pentyl.

Cycloalkyl-$C_1$-$C_7$-alkyl $R_3$ ist beispielsweise 2-Cycloalkyl-$C_1$-$C_7$-alkyl, z.B. Cycloprop-2-ylprop-2-yl, Cyclopent-2-ylprop-2-yl oder Cyclohex-2-ylprop-2-yl.

Aryl $R_3$ ist beispielsweise Phenyl oder Naphthyl.

Triaryl-$C_1$-$C_7$-alkyl $R_3$ ist beispielsweise Triphenylmethyl.

$R_3$ ist bevorzugt tert-$C_4$-$C_7$alkyl, insbesondere tert-Butyl, oder Phenyl.

Eine Carboxyschutzgruppe $R_3'$ ist in den unter Hydroxyschutzgruppen $R_2$ genannten Standardwerken beschrieben und ist beispielsweise eine Estergruppe, welche unter schonenden Bedingungen selektiv abspaltbar ist, beispielsweise Niederalkyl, z.B. Methyl oder Ethyl, Arylmethyl mit einem oder zwei Arylresten, worin Aryl vorzugsweise unsubstituiertes oder z.B. durch Niederalkyl, z.B. tert-Niederalkyl, z.B. tert-Butyl, Niederalkoxy, z.B. Methoxy, Hydroxy, Halogen, z.B. Chlor, und/oder Nitro mono-, di- oder trisubstituiertes Phenyl bedeutet, beispielsweise Benzyl durch die genannten Substituenten substituiertes Benzyl, z.B. 4-Nitrobenzyl oder 4-Methoxybenzyl, Diphenylmethyl oder die genannten Substituenten substituiertes Diphenylmethyl, z.B. Di-(4-methoxyphenyl)-methyl, sowie 1-Niederalkoxyniederalkyl, z.B. Methoxymethyl, 1-Methoxyethyl oder 1-Ethoxymethyl, 1-Niederalkylthioniederalkyl, z.B. 1-Methylthiomethyl oder 1-Ethylthioethyl, Aroylmethoxycarbonyl, z.B. Phenacyloxycarbonyl, sowie 2-Halogenniederalkyl, z.B. 2,2,2-Trichlorethyl, 2-Bromethyl oder 2-Jodethyl.

$R_3'$ ist bevorzugt tert-$C_1$-$C_7$-Alkyl, z.B. tert-Butyl, Phenyl, 4-Nitrobenzyl oder 4-Methoxybenzyl.

Eine Aminoschutzgruppe $R_4'$ ist beispielsweise acidolytisch abspaltbares, in 1-Stellung des Niederalkylrests verzweigtes, Niederalkoxycarbonyl, reduktiv, z.B. hydrogenolytisch, abspaltbares Arylmethoxycarbonyl, worin Aryl durch eine oder zwei Nitrogruppen substituiert sein kann, oder ist bevorzugt oxydativ abspaltbares Arylmethyl oder Aryl, worin Aryl bevorzugt durch eine oder zwei Niederalkoxygruppen, z.B. Methoxy, substituiert ist, ferner eine oxydativ abspaltbare 2-Niederalkenylgruppe.

In 1-Stellung des Niederalkylrests verzweigtes Niederalkoxycarbonyl $R_4'$ ist beispielsweise tert-Niederalkoxycarbonyl, z.B. tert-Butoxycarbonyl (BOC). Ebenso geeignet sind analoge Gruppen, z.B. tert-Amyloxycarbonyl, Isopropoxycarbonyl, Cyclohexyloxycarbonyl, d-Isobornyloxycarbonyl oder Adamantyloxycarbonyl.

Arylmethoxycarbonyl $R_4'$ , worin Aryl durch eine oder zwei Nitrogruppen substituiert sein kann, ist z.B. Benzyloxycarbonyl, 4-Nitrobenzyloxycarbonyl oder 2,4-Dinitrobenzyloxycarbonyl.

Eine oxydativ abspaltbare Niederalkenylgruppe $R_4'$ ist beispielsweise Allyl.

Eine Aminoschutzgruppe $R_4'$ ist vorzugsweise oxydativ abspaltbares Arylmethyl oder Aryl $R_4'$ , worin Aryl bevorzugt durch eine oder zwei Niederalkoxygruppen, z.B. Methoxy, substituiertes Phenyl ist, insbesondere 4-Methoxybenzyl oder 2,4-Dimethoxybenzyl bzw. 4-Methoxyphenyl oder 3,4-Dimethoxyphenyl.

In einem Ausgangsmaterial der Formel (III) ist eine unter den Bedingungen des Ringschlussverfahrens nicht abspaltbare Hydroxyschutzgruppe $R_2'$ eine der genannten Acylgruppen einer substituierten Carbon- oder Sulfonsäure, z.B. 2,2,2-Trifluoracetyl, die substituierte Acylgruppe eines Kohlensäurehalbesters, z.B. p-Nitrobenzyloxycarbonyl, 2-Tetrahydrofuryl oder -pyranyl, oder 1-Niederalkoxyniederalkyl, z.B. Methoxymethyl oder 1-Ethoxyethyl.

In einem Ausgangsmaterial der Formel (III) ist eine geeignete nukleofuge Abgangsgruppe X beispielsweise Halogen, z.B. Chlor, Brom oder Jod, Niederalkanoyloxy, z.B. Acetoxy, Arylsulfonyloxy, z.B. Phenylsulfonyloxy oder Tosyloxy, oder Niederalkylsulfonyloxy, z.B. Mesyloxy.

Als Tetra-$C_1$-$C_7$-alkylammoniumfluorid ist z.B. Tetra-n-butylammoniumfluorid zu nennen.

Die Zyklisierung wird in einem aprotischen, organischen Lösungsmittel unter Ausschluß von Wasser durchgeführt, z.B. in einem Ether, z.B. Diethylether, Dioxan oder Tetrahydrofuran, einem Amid, z.B. Dimethylformamid oder Hexamethylphosphorsäuretriamid, oder in einer Mischung davon, gegebenenfalls auch einer Mischung der genannten Lösungsmittel mit einem Alkan, z.B. n-Hexan oder Petrolether. Die Reaktionstemperatur liegt zwischen -10° und Raumtemperatur. Man arbeitet vorzugsweise unter Inertgasatmosphäre, z.B. Argon- oder Stickstoffatmosphäre. Bei Verwendung von wasser haltigem Tetra-n-butylammoniumfluorid wird das Wasser durch ein übliches Trocknungsmittel, vorzugsweise durch Zugabe zur Reaktionsmischung von frisch aktiviertem Molekularsieb, entzogen, z.B. mit 4 Å Porendurchmesser.

Bei dem erfindungsgemässen Verfahren findet eine Inversion der Konfiguration am 2-C-Atom des Ausgangsmaterials statt, so dass aus einer 2R-Verbindung der Formel (II) oder (III) die 3S-Verbindung der Formel (I) entsteht. Die Konfiguration des 1'-C-Atoms ($R_1$ = Niederalkyl) in der Seitenkette bleibt unverändert. Beispielsweise erhält man aus R-Glycidsäureamiden der Formel (II) 3-(S)-Azetidinone der Formel (I) oder aus (2 R,3 R)-2,3-Epoxybuttersäureamiden der Formel (II) 3-(S)-(1-(R)-hydroxyethyl)-azetidinone der Formel (I).

Wenn die Herstellung von Verbindungen der Formel (I) beabsichtigt ist, worin $R_1$ $C_1$-$C_7$ Alkyl, z.B. Methyl, bedeutet, werden vorzugsweise Verbindungen der Formeln II oder III verwendet, welche am 2- und am 3-C-Atom die R-Konfiguration haben.

Die vorliegende Erfindung hat vorzugsweise ein Verfahren zur Herstellung von Verbindungen der Formel (I) zum Gegenstand, worin $R_1$ Wasserstoff oder Methyl, $R_2$ Wasserstoff oder eine Hydroxyschutzgruppe, z.B. die substituierte Acylgruppe eines Kohlensäurehalbesters, z.B. 2,2,2-Trichlorethoxycarbonyl, Allyloxycarbonyl, Benzyloxycarbonyl oder p-Nitrobenzyloxycarbonyl oder Triniederalkylsilyl, z.B. Trimethylsilyl oder Dimethyl-tert-butylsilyl, Y die Gruppen $-S(=O)_2-R_3$ oder $-C(=O)-O-R_3'$ , worin $R_3$ bzw. $R_3'$ tert-$C_4$-$C_7$-alkyl, z.B. tert-Butyl, oder Phenyl bedeuten, und $R_4$ Wasserstoff oder eine Aminoschutzgruppe $R_4'$ , z.B. 4-Methoxybenzyl, 2,4-Dimethoxybenzyl, 4-Methoxyphenyl oder 3,4-Dimethoxyphenyl, bedeutet, und worin das 4-C-Atom des Azetidinonrings die R-Konfiguration hat, wenn Y die Gruppe $-S(=O)_2-R_3$ bedeutet, und die S-Konfiguration hat, wenn Y die Gruppe $-C(=O)-O-R_3'$ bedeutet, und das 1'-C-Atom der Seitenkette die R-oder die S-Konfiguration hat, wenn $R_1$ $C_1$-$C_7$-Alkyl bedeutet.

In einer bevorzugten Ausführungsform des Verfahrens werden Ausgangsmaterialien der Formel (II) verwendet, worin $R_1$ Wasserstoff oder Methyl, Y die Gruppen $-S(=O)_2-R_3$ oder $C(=O)-O-R_3'$ , worin $R_3$ bzw. $R_3'$ tert-$C_1$-$C_7$-alkyl, z.B. tert-Butyl, oder Phenyl bedeuten, und $R_4'$ eine Aminoschutzgruppe, z.B. 4-Methoxybenzyl, 2,4-Dimethoxybenzyl, 4-Methoxyphenyl oder 3,4-Dimethoxyphenyl, bedeutet, und das 1'-C-Atom der Seitenkette die R- oder die S-Konfiguration hat, wenn $R_1$ Methyl bedeutet, welche man vorzugsweise aus Verbindungen der Formel (IV)

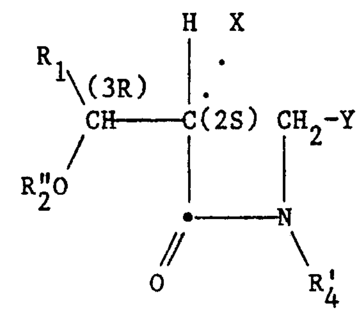

(IV);

gegebenenfalls in situ herstellt, worin Y, $R_1$ und $R_3$ die genannten Bedeutungen haben, $R_2''$ Wasserstoff oder eine unter den Bedingungen der Epoxidbildung abspaltbare Hydroxyschutzgruppe, z.B. eine Triniederalkylsilylgruppe, $R_4'$ eine Aminoschutzgruppe und X eine nukleofuge Abgangsgruppe ist, und worin das 3-C-Atom die R-Konfiguration hat, wenn $R_1$ Methyl bedeutet.

In einer erhältlichen Verbindung der Formel (I) erfolgt die Abspaltung der Aminoschutzgrupe $R_4'$ in an sich bekannter Weise, z.B. durch Solvolyse, z.B. Acidolyse, Reduktion oder Oxydation.

So kann z.B. tert-Niederalkoxycarbonyl $R_4'$ durch Behandeln mit einer geeigneten Säure, z.B. Ameisen- oder Trifluoressigsäure, gegebenenfalls in Gegenwart von Wasser, abgespalten werden.

Arylmethoxycarbonyl $R_4'$ , z.B. Benzyloxycarbonyl, 4-Nitrobenzyloxycarbonyl oder 2,4-Dinitrobenzyloxycarbonyl, kann z.B. durch Behandeln mit einem geeigneten Reduktionsmittel, z.B. Zink, in Gegenwart einer Carbonsäure, z.B. Essigsäure, abgespalten werden.

Eine Arylmethylgruppe $R_4'$ , z.B. 4-Methoxy- oder 2,4-Dimethoxybenzyl, wird z.B. oxydativ abgespalten, z.B. durch Umsetzung mit einem starken Oxydationsmittel, z.B. einem anorganischen Peroxidsalz, z.B. Natrium- oder Kaliumperoxodisulfat, in Gegenwart eines sauren Salzes, z.B. Dikaliumhydrogenphosphat, und gegebenenfalls in Gegenwart eines Katalysators, z.B. eines Metallsalzes, z.B. eines Eisen(II)- und/oder Kupfer(II)-salzes, z.B. Eisen(II)-sulfatheptahydrat und/oder Kupfer(II)-acetathydrat oder bevorzugt oxydativ durch Umsetzung mit einem Cer(IV)-Salz, z.B. Cer(IV)iodat, Cer(IV)-nitrat ($CeOH(NO_3)_3$), Cer(IV)-sulfat oder, bevorzugt, Diammonium Cer(IV)-hexanitrat, in Gegenwart einer Säure, z.B. wässriger HCl, und anschliessende Umsetzung mit einem Reduktionsmittel, z.B. Natriumbisulfit.

Eine Arylgruppe $R_4'$ , z.B. 4-Methoxyphenyl oder 3,4-Dimethoxyphenyl, ferner die Niederalkenylgruppe, wird bevorzugt oxydativ abgespalten, z.B. ozonolytisch in Ethylacetat und anschliessende Zersetzung der gebildeten Ozonide durch Reduktion mit einem Reduktionsmittel, z.B. Natriumthiosulfat, oder durch Umsetzung mit einem Cer(IV)-Salz, z.B. Diammonium-Cer(IV)-hexanitrat, und anschliessende Reduktion.

Die beschriebenen Spaltungsreaktionen werden unter an sich bekannten Bedingungen durchgeführt, wenn notwendig unter Kühlen oder Erwärmen und gegebenenfalls in einer Inertgas-, z.B. Stickstoffatmosphäre.

Eine Hydroxyschutzgruppe $R_2$ kann in an sich bekannter Weise eingeführt werden, z.B. durch Umsetzung einer Verbindung der Formel (I), worin $R_2$ Wasserstoff bedeutet, mit einem reaktionsfähigen Derivat einer Säure, dessen Acylgruppe die Hydroxyschutzgruppe ist, z.B. durch Umsetzung mit einem Anhydrid, z.B. Trifluoressigsäureanhydrid, einem gemischten Anhydrid, z.B. einem Säurehalogenid, z.B. 2,2-Dichloracetylchlorid, einem Anhydrid eines Kohlensäurehalbesters, z.B. 2,2,2-Trichlorethoxy-, Phenyl- oder p-Nitrophenylchloroformiat, durch Reaktion mit 1,2-Dihydrofuran oder 1,2-Dihydropyran in Gegenwart einer Säure, z.B. p-Toluolsulfonsäure, oder durch Reaktion mit einem Triniederalkylhalogensilan, z.B. Trimethylchlor- oder Dimethyl-tert-butylchlorsilan.

Eine erhältliche Verbindung der Formel (I), worin Y die Gruppe $-S(=O)-R_3$ bedeutet, kann z.B. mit einem geeigneten Oxydationsmittel in die entsprechende Verbindung der Formel (I), worin Y die Gruppe $S-(=O)_2-R_3$ bedeutet, umgewandelt werden.

Ein geeignetes Oxydationsmittel ist beispielsweise Wasserstoffperoxid, eine Persäure, z.B. eine aliphatische oder aromatische Persäure, z.B. Peressigsäure, Perbenzoesäure oder m-Chlorperbenzoesäure, sowie die anodische Oxydation, z.B. an einer Platinelektrode, in saurer, z.B. schwefelsaurer, Lösung. Die Oxydation wird bevorzugt in einem geeigneten inerten Lösungsmittel, beispielsweise in einem Halogenkohlenwasserstoff z.B. Methylenchlorid oder Chloroform, einem Alkohol, z.B. Methanol oder Ethanol, einem Keton, z.B. Aceton, einem Ether, z.B. Diethylether, Dioxan oder Tetrahydrofuran, einer flüssigen organischen Carbonsäure, z.B. Essigsäure, oder in einem wässrigen Gemisch dieser Lösungsmittel, bei Raumtemperatur, unter Kühlen oder Erwärmen, z.B. bei etwa -20° C bis ca. +90° C, bevorzugt bei -20° C bis etwa 30° C, durchführt.

Die Weiterverarbeitung von Verbindungen der Formel (I) ($R_4$ = H) erfolgt in an sich bekannter Weise und führt, je nach Bedeutung von Y zu (5R,6S)-Penem-3-carbonsäuren oder (5R,6S)-Carbapenemen mit wertvollen pharmakologischen, z.B. antibakteriellen, Eigenschaften.

Die Weiterverarbeitung einer Verbindung der Formel (I), worin Y die Gruppe -C(=O)-O-$R_3'$ darstellt und $R_4'$ eine Aminoschutzgruppe ist, kann auf zwei verschiedenen Wegen erfolgen. Man kann zunächst die Aminoschutzgruppe $R_4'$ , z.B. p-Methoxybenzyl, entfernen, z.B. wie weiter vorn geschildert durch Oxydation mit Cer(IV)-ammoniumnitrat, und dann die Gruppe Y durch übliche Spaltung der Estergruppe in die freie Carbonsäure -C(=O)-OH, z.B. durch Einwirkung von Trifluor essigsäure, überführen und diese Carbonsäure mit Blei(IV)-acetat in einem inerten Lösungsmittel oxydativ in ein Azetidinon umwandeln, das in 4-Stellung durch Acetoxy substituiert ist.

Man kann auch in umgekehrter Reihenfolge der Reaktionsschritte vorgehen, indem man zuerst die Estergruppe $R_3'$ abspaltet und die freie Carbonsäure mit Blei(IV)-acetat oxydativ unter $CO_2$-Abspaltung zur 4-Acetoxy-Verbindung umsetzt, welche man selektiv in trans-Form erhält. In einem weiteren Schritt wird die Aminoschutzgruppe $R_4'$ wie beschrieben mit Cer(IV)-ammoniumnitrat abgespalten. Azetidinone, die in 4-Stellung durch Acetoxy substituiert sind, können anschliessend zu Penemen oder Carbapenemen weiterverarbeitet werden.

Die Herstellung der Ausgangsmaterialien der Formeln (II) und (III) erfolgt in an sich bekannter Weise, z.B. analog zu den von Yanagisawa H. et al. oder Shiozaki M. et al. beschriebenen Verfahren.

Beispielsweise kann man aus einer Verbindung der Formel (IV), worin Y, $R_1$ und $R_3$ die unter Formel (I) genannten Bedeutungen haben, $R_2''$ Wasserstoff oder eine unter den Bedingungen der Epoxidbildung abspaltbare Hydroxyschutzgruppe, $R_4'$ eine Aminoschutzgruppe und X eine nukleofuge Abgangsgruppe darstellt, die Säure HX abspalten und eine erhältliche Verbindung, worin Y die Gruppe -S-$R_3$ darstellt, in eine Verbindung, worin Y die Gruppen C(=O)-O-$R_3$ oder -$SO_2$-$R_3$ darstellt umwandeln.

In einer Verbindung der Formel (IV) ist eine unter den Bedingungen der Epoxidbildung abspaltbare Hydroxyschutzgruppe $R_2''$ eine Triniederalkylsilylgruppe, z.B. Dimethyl-tert-butylsilyl oder Trimethylsilyl. X bedeutet bevorzugt Halogen, z.B. Chlor oder Brom, Niederalkylsulfonyloxy, z.B. Mesyloxy, oder Arylsulfonyloxy, z.B. Tosyloxy.

Die Abspaltung von HX erfolgt mit einer der unter Verfahren b) genannten Basen, z.B. Natrium- oder Kaliumhydroxid oder Natrium-oder Kaliumcarbonat, vorzugsweise mit einem Amidin, z.B. 1,5-Diazabicyclo [5.4.0]undec-5-en, oder mit einem Tetraniederalkylammoniumfluorid, z.B. entwässertem Tetra-n-butylammoniumfluorid.

Die Umsetzung zu einer Epoxyverbindung der Formel (II) wird vorzugsweise in einem inerten, vorzugsweise wasserfreien, Lösungsmittel oder Lösungsmittelgemisch vorgenommen, beispielsweise in einem Carbonsäureamid, z.B. Dimethylformamid, einem halogenierten Kohlenwasserstoff, z.B. Methylenchlorid, Chloroform, Tetrachlorkohlenstoff oder Chlorbenzol, einem Keton, z.B. Aceton, cyclischen Aether, z.B. Dioxan oder Tetrahydrofuran, einem Ester, z.B. Essigsäureethylester, oder einem Nitril, z.B. Acetonitril oder in Mischungen davon, gegebenenfalls bei erniedrigter oder erhöhter Temperatur, z.B. in einem Temperaturbereich von etwa -40 °C bis etwa +100 °C, bevorzugt von etwa -10 °C bis etwa +50 °C, und gegebenenfalls unter Inertgas-, z.B. Stickstoffatmosphäre.

Bei der Umsetzung gemäss Verfahren c) kehrt sich die Konfiguration des 2-C-Atoms um, während die Konfiguration des 3-C-Atoms ($R_1$ = Niederalkyl) erhalten bleibt. Aus 2-(S)-Halogen-3-(R)-hydroxycarbonsäureamiden der Formel IV entstehen die (2R,3R)-Verbindungen der Formel (II). Entsprechendes gilt für die 2-(S)-Halogen-3-(S)-hydroxycarbonsäureamide der Formel (II).

Die Umwandlung einer Verbindung der Formel (IV) in die Epoxyverbindung der Formel (II) erfolgt in an sich bekannter Weise, z.B. unter den aus der Steroidchemie, siehe Djerassi C. et al. "Steroid Reactions", Holden Day, San Francisco 1963, S. 606-613, bekannten Reaktionsbedingungen.

Verbindungen der Formel (IV) lassen sich beispielsweise durch Amidierung einer Carbonsäure der Formel (V)

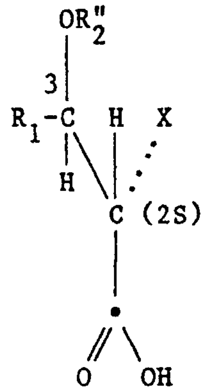

(V)

worin $R_1$ die unter Formel I und $R_2''$ die unter Formel IV genannte Bedeutung hat und X eine nukleofuge Abgangsgruppe darstellt, mit einem Amin der Formel (VI)

$$R_4'NH-CH_2-Y' \qquad (VI).$$

worin Y' die Gruppe $-S-R_3$, $-S(=O)-R_3$, $-S(=O)_2-R_3$ oder $-C(=O)-O-R_3'$ bedeutet und $R_4'$ eine Aminoschutzgruppe ist, oder durch Kondensation eines Amids der Formel (VII)

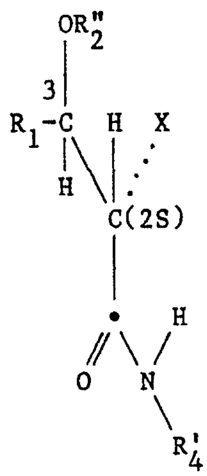

(VII),

worin $R_1$ die unter Formel I und $R_2''$ die unter Formel IV genannte Bedeutung hat, X eine nukleofuge Abgangsgruppe und $R_4'$ eine geeignete Aminoschutzgruppe darstellt, mit einem Halogenid der Formel

$$Z-CH_2-Y' \qquad (VIII),$$

worin Z Halogen, z.B. Chlor, Brom oder Jod ist, und gegebenenfalls Oxydation der Gruppe $-S-R_3$, herstellen.

Verbindungen der Formel (V) sind bekannt. Diese lassen sich beispielsweise in an sich bekannter Weise aus L-Serin oder L-Threonin durch Diazotierung der Aminogruppe mit einem Nitritsalz, z.B. Kaliumnitrit und Umsetzung der erhaltenen Diazoverbindung mit einem Bromidsalz, z.B. Kaliumbromid, herstellen.

Bei Verwendung von L-Serin lassen sich nach den beschriebenen Verfahren ausgehend von Verbindungen der Formel (V) Verbindungen der Formel (I) erhalten, worin der Kohlenstoff in 3-Stellung des Azetidinonrings die S-Konfiguration hat.

Bei Verwendung von L-Threonin erhält man Verbindungen der Formel (I), worin das C-Atom in 3-Stellung des Azetidinonrings die S-Konfiguration und das 1'-C-Atom der Hydroxyethyl-Seitenkette die R-Konfiguration hat. Ist die Herstellung von Verbindungen der Formel (I) beabsichtigt, worin das 3-C-Atom die S-Konfiguration und das 1'-C-Atom der Hydroxyethyl-Seitenkette die R-Konfiguration hat, geht man von Allothreonin aus. Bei allen beschriebenen Umsetzungen bleibt die Konfiguration des C-Atoms, welches in Verbindungen der Formel (I) dem 1'-C-Atom der 3-Niederalkyl-CH-($OR_2$)-Seitenkette entspricht, unverändert.

Verbindungen der Formel (VII) sind bekannt oder lassen sich, falls sie neu sind, in an sich bekannter Weise nach den Vorschriften gemäss Reynolds D.D. und Cossar B.C., J.Heterocycl. Chem. 8 , 597 (1971) herstellen.

Die vorliegende Erfindung hat auch solche Ausführungsformen des Verfahrens zum Gegenstand, in denen man von einer auf irgendeiner Vorstufe des Verfahrens erhältlichen Verbindung ausgeht und die anschliessenden Verfahrensschritte ausführt.

Die folgenden Beispiele dienen zur Illustration der Erfindung. Temperaturen sind in Graden Celsius, und IR-Werte in $cm^{-1}$ angegeben. Für die $^1$H-NMR-Spektren sind die chemischen Verschiebungen ($\delta$) in ppm und Kopplungskonstanten J in Herz (Hz) angegeben. Die spezifischen Drehwerete [$\alpha$] sind $[\alpha]_D^{20}$ -Werte. Für die chromatographische Trennung bei der präparativen Aufarbeitung lassen sich Säulen gefüllt mit Merck-Kieselgel 60 verwenden. Rf-Werte gelten für Fertigplatten für die Dünnschichtchromatographie (Merck 60 F 254). Abkürzungen:

m = mittelstarke Absorptionsbande
sh = scharfe Absorptionsbande
S = Singulett
M = Multiplett
D = Dublett
F. = Schmelzpunkt
Kp. = Siedepunkt

Beispiel 1: Benzyl-tert-butylthiomethylammoniumchlorid (Ausgangsprodukt)

Die Verbindung wird analog einer allgemeinen Vorschrift von D.D. Reynolds und B.C. Cossar, J.Heterocycl. Chem. 8 , 597 (1971) folgendermassen hergestellt:

Durch Zugabe von 1,19 g (3,33 mMol) 1,3,5-Tribenzylhexahydro-1,3,5-triazin in 5 ml Diethylether zu 15 ml einer Lösung von 0,40 g Chlorwasserstoff in Ether bei -20° entsteht die Suspension eines weissen, kristallinen Niederschlages, die dann bei derselben Temperatur, mit 1,19 ml (10,5 mMol) tert-Butylmercaptan versetzt wird. Man rührt bei Raumtemperatur unter Feuchtigkeitsausschluss weiter, wobei sich die ursprünglichen Kristalle vorübergehend zusammenballen und neue, weisse Kristalle der Titelverbindung gebildet werden. Diese werden nach 4 Stunden Rühren abgesaugt, mit etwas Diethylether gewaschen und im Vakuum getrocknet. F. 107° (Umwandlung zu neuen Kristallen; total geschmolzen bei 142°).

Das Ausgangsmaterial 1,3,5-Tribenzylhexahydro-1,3,5-triazin kann ebenfalls gemäss D.D.Reynolds und B.C. Cossar, supra, hergestellt werden. Diese Verbindung hat folgende charakteristische Daten:

IR (in $CH_2Cl_2$): 3080-2600 (m), 1600, 1490, 1450, 1360, 1340 (sh), 1312, 1168, 1117, 1071, 1030, 1016, 980, 920; $^1$H-NMR (60 MHz, in $CDCl_3$): $\delta$ = 3,48 (S,2H), 3,70 (S,2H), 7.36 (M,5H).

Beispiel 2: N-Benzyl-N-tert-butylthiomethyl-2(S)-brom-3-hydroxypropionsäureamid (Ausgangsprodukt)

Zu einem Gemisch von 338 mg (2 mMol) 2-(S)-Brom-3-hydroxypropionsäure und 492 mg (2 mMol) N-Benzyl-N-tert-butylthiomethylammonium chlorid in 10 ml absolutem Tetrahydrofuran wird unter Rühren und Argonatmosphäre bei Raumtemperatur 202 mg (2 mMol) Triethylamin und 413 mg (2 mMol) Dicyclohexylcarbodiimid gegeben und das entstandene Reaktionsgemisch bei Raumtemperatur 2 Stunden weitergerührt. Der ausgeschiedene Dicyclohexylharnstoff wird abgenutscht und mit etwas THF nachgewaschen. Die vereinigten Filtrate werden unter vermindertem Druck eingedampft, und der ölige Rückstand an einer Kieselgelsäule mit Toluol: Ethylacetat (9:1) chromatographiert. Nach Abnahme der ersten Fraktionen, die kleine Mengen von N,N-Bis-(tert-butylthiomethyl)-benzylamin enthalten, wird die Titelverbindung als farbloses, beim Stehen langsam kristallisierendes Oel eluiert. F. 65-68° (aus Hexan); Rf (Toluol: Ethylacetat 1:1): 0,56. Das $^1$H-NMR-Spektrum (in $CDCl_3$ und in $D_6$-Benzol) deutet auf das Vorhandensein von zwei Rotameren (2 Gruppen von Signalen).

Das bekannte Ausgangsmaterial 2(S)-Brom-3-hydroxypropionsäure kann gemäss der Vorschrift von U. Shimohigashi, M. Waki und N. Izumiya, Chem. Abstr. 91, 39804 u (1979) hergestellt werden.

Beispiel 3: N-Benzyl-N-tert-butylsulfinylmethyl-2-(S)-brom-3-hydroxypropionsäureamid (Ausgangsprodukt)

Eine Lösung von 90 mg (0,25 mMol) N-Benzyl-N-tert-butylthiomethyl-2-(S)-brom-3-hydroxypropionsäureamid in 5 ml Methylenchlorid wird bei -20° langsam mit 48 mg (ca. 0,25 mMol) 90 %iger m-Chlorper benzoesäure, gelöst in 5 ml Methylenchlorid, versetzt und das entstandene Reaktionsgemisch eine Stunde bei -20° weitergerührt. Nach Waschen mit einer 5 %igen wässrigen $NaHCO_3$-Lösung wird die organische Schicht über wasserfreiem $Na_2SO_4$ getrocknet und eingedampft. Der ölige Rückstand enthält die Titelverbindung in Form von zwei isomeren S-Oxiden. Rf (Toluol-Ethylacetat 1:1): 0,13-0,20 (überlappend); IR ($CH_2Cl_2$): 3560-3250, 2950, 1654, 1495, 1470-1390, 1362, 1230-1140, 1040, 910.

Beispiel 4: N-Benzyl-N-tert-butylsulfonylmethyl-2-(S)-brom-3-hydroxypropionsäureamid (Ausgangsprodukt)

Zu einer Lösung von 3,44 g (9,56 mMol) N-Benzyl-N-tert-butylthiomethyl-2-(S)-brom-3-hydroxypropionsäureamid in 75 ml Methylenchlorid wird bei -10° unter Rühren insgesamt 4,03 g (ca. 21 mMol) 90 %ige m-Chlorperebenzoesäure in mehreren Portionen innerhalb von 15 Miuten gegeben. Nach einer weiteren Stunde Rühren bei 0° wird das Reaktionsgemisch mit 100 ml Methylenchlorid verdünnt und nacheinander mit 75 ml einer 2 %igen wässrigen $NaHSO_3$-Lösung und mit 75 ml einer 8 %igen wässrigen $NaHCO_3$-Lösung gewaschen. Die wässrigen Anteile werden mit 50 ml Methylenchlorid nachextrahiert. Nach Trocknen der vereinigten organischen Phasen über Natriumsulfat und Eindampfen im Vakuum bleibt die Titelverbindung als beinahe farbloses Oel zurück. Rf (Toluol: Ethylacetat 1:1): 0,36; IR ($CH_2Cl_2$): 3600-3350, 3000-2850, 1661, 1494, 1475 (sh), 1450, 1420, 1395, 1352, 1300, 1250 (sh), 1220, 1197, 1173, 1140, 1110, 1035, 880.

Beispiel 5: N-Benzyl-N-tert-butylthiomethyl-(R)-glycidsäureamid (Ausgangsprodukt)

Eine Lösung von 845 mg (5 mMol) 2-(S)-Brom-3-hydroxypropionsäure in 10 ml absolutem Tetrahydrofuran wird bei Raumtemperatur unter Rühren mit 5 ml einer 2M-Lösung von 1,5-Diazabicyclo[5.4.0]undec-5-en (DBU) in Tetrahydrofuran versetzt und das entstandene Gemisch 2 Stunden bei Raumtemperatur weitergerührt. Das Lösungsmittel wird nachher im Vakuum abdestilliert und 25 ml Methylenchlorid zugegeben. Zu dieser Lösung, enthaltend das DBU-Salz der (R)-Glycidsäure, wird 1,03 g (5 mMol) Dicyclohexylcarbodiimid und nach einer Stunde Rühren 1,3 g (5 mMol) N-Benzyl-N-tert-butylthiomethylammoniumchlorid zugegeben. Nach Zugabe der letztgenannten Reaktionskomponente bilded sich kristalliner Dicyclohexylharnstoff. Dieser wird nach 3 Stunden Reaktionszeit bei Raumtemperatur abgesaugt, das Filtrat mit Methylenchlorid verdünnt und mit Phosphatpufferlösung vom pH 8 gewaschen. Nach Eindampfen der organischen Phase wird der Rückstand an 50 g Kieselgel chromatographiert. Nach Abnahme von einigen ersten Fraktionen mit Toluol wird die Titelverbindung mit einem Gemisch aus Toluol-Ethylacetat (9:1) als farbloses, viskoses Oel eluiert. Rf (Toluol: Ethylacetat 1:1): 0,45; $[\alpha] = +24 \pm 1°$ (1,12 % in Chloroform). Das $^1H$-NMR-Spektrum (in $CDCl_3$) weist zwei Gruppen von Signalen auf, die zwei Rotameren im Verhältnis von ca. 2:1 entsprechen.

Beispiel 6: N-Benzyl-N-tert-butylsulfonylmethyl-(R)-glycidsäureamid (Ausgangsprodukt)

Zu einer Lösung von 910 mg (3,26 mMol) N-Benzyl-N-tert-butylthiomethyl-(R)-glycidsäureamid in 30 ml Methylenchlorid wird bei -10° in mehreren Portionen insbesamt 1,37 g (ca. 7,12 mMol) 90 %ige m-Chlorperbenzoesäure zugegeben und das resultierende Gemisch bei 0° 90 Minuten weitergerührt. Danach wird das Reaktionsgemisch mit etwas $CH_2Cl_2$ versetzt und mit kalter, wässriger 2 %iger Natriumbisulfit-Lösung gewaschen. Das in der $CH_2Cl_2$-Phase enthaltene Rohprodukt wird an 50 g Kieselgel chromatographiert. Mit einem Gemisch aus Toluol-Ethylacetat (1:1) wird die kristalline Titelverbindung eluiert. F. 106-107° (aus Methylenchlorid-Diethylether); Rf (Toluol: Ethylacetat 1:1): 0,29; $[\alpha] = +24 \pm 1°$ (1,31 % in $CHCl_3$). $^1H$-NMR-Spektrum (in $CDCl_3$): 2 Gruppen von Signalen deuten auf 2 Rotamere im Verhältnis von ca. 4:1.

Beispiel 7: N-Benzyl-N-tert-butylsulfonylmethyl-(R)-glycidsäureamid (Ausgangsprodukt)

Eine Lösung von 118 g (3 mMol) N-Benzyl-N-tert-butylsulfonylmethyl-2-(S)-brom-3-hydroxypropionsä ureamid (siehe Beispiel 4) in 15 ml absolutem Tetrahdrofuran wird bei 0° mit 1,125 g (7,4 mMol) 1,5-Diazabicyclo[5.4.0]undec-5-en versetzt und bei Raumtemperatur 90 Minuten weitergeführt. Nach Aufarbeiten mit Methylenchlorid und Phosphatpuffer vom pH 7,0 wird das aus der organischen Phase erhaltene Rohprodukt an 50 g Kieselgel chromatographiert. Nach Eluieren mit einem Gemisch von Ethylacetat-Toluol (1:1) erhält man die mit der Verbindung gemäss Beispiel 6 identische, reine Titelverbindung.

Beispiel 8: 1-Benzyl-(3)-S-hydroxymethyl-4-(R)-tert-butylsulfonyl-2-azetidinon

a) Eine Lösung von 103,9 mg (0.333 mMol) N-Benzyl-N-tert-butylsulfonylmethyl-R-glycidsäureamid in 0,5 ml absolutem Tetrahydrofuran wird bei 0° mit 3 ml einer Tetra-n-butylammoniumfluorid-Lösung versetzt, welche aus 5,0 g Tetra-n-butylammoniumfluorid-trihydrat (Fluka) durch Trocknen bei 60°/0,1 Torr und Auffüllen mit absolutem Tetrahydrofuran auf 20 ml hergestellt worden ist. Nach einer Stunde Rühren bei 0° wird das Reaktionsgemisch in einem 5:1-Gemisch von Diethylether und Methylenchlorid aufgenommen und mit wässrigem Phosphatpuffer, pH 8,0 gewaschen. Nach Trocknen der organischen Phase mit wasserfreiem Natriumsulfat und Eindampfen im Vakuum wird durch Verreiben mit etwas Ether die reine Titelverbindung erhalten. F. 139-141°; Rf (Ethylacetat:Hexan 2:1): 0,17; [α] = +35 ± 1° (1.30 % in $CHCl_3$); $^1$H-NMR-Spectrum: δ = 4,71 (D) für Proton (a) am 4(R)-C-Atom und δ = 3,75 (M) für Proton (b) am 3(S)-C-Atom (trans-Verbindung), J a-b: ca. 2.

b) Eine Lösung von 196 mg (0,5 mMol) N-Benzyl-N-tert-butylsulfonylmethyl-2-(S)-brom-3-hydroxypropionsäureamid (Beispiel 4) in 0,8 ml Tetrahydrofuran wird mit 5,9 ml einer Lösung versetzt, die man aus 5 g Tetra-n-butylammoniumfluorid-trihydrat durch Entwässern bei 55-60°/0,1 mm und Auflösen in 20 ml Tetrahydrofuran herstellt. Das erhaltene Reaktionsgemisch wird nach Zugabe von aktiviertem Molekularsieb (Typ 4A 1/16, Fa. Dr. Bender & Dr. Hobein) zwei Stunden bei +5° gerührt. Das Molekularsieb wird abgenutscht und mit Methylenchlorid gewaschen. Die vereinigten Filtrate werden mit Ether verdünnt und mit Phosphatpuffer vom pH 8 gewaschen. Nach Trocknen der organischen Phase über Natriumsulfat und Eindampfen unter vermindertem Druck bleibt ein Rohprodukt zurück, welches durch Chromatographieren an präparativen Kieselgelplatten mit Hexan-Ethylacetat (1:3) die kristalline Titelverbindung ergibt.

Beispiel 9: N-Allyl-N-tert-butylthiomethylammoniumchlorid (Ausgangsprodukt)

Die Titelverbindung wird analog einer allgemeinen Vorschrift von D.D. Reynolds und B.C. Cossar, J.Heterocycl. Chem. 8 ,597 (1971), folgendermassen hergestellt:

Zu einer Lösung von 10,8 g (0,3 Mol) Chlorwasserstoff in 100 ml Ether wird bei -20° eine Lösung von 18,65 g (0,09 Mol), 1,3,5-Triallylhexahydro-1,3,5-triazin in 175 ml Ether zugetropft, die entstandene Suspension mit 30,6 ml (0,27 Mol) tert-Butylmercaptan versetzt und das Gemisch 18 Stunden bei Raumtemperatur weitergerührt.

Die ausgeschiedenen, weissen Kristalle werden abgenutscht, mit Ether gewaschen und aus Methylenchlorid-Ether umkristallisiert. Es werden weisse Kristalle der Titelverbindung, F. 85° - 90°, erhalten.

Das Ausgangsmaterial 1,3,5-Triallylhexahydro-1,3,5-triazin kann nach der Vorschrift von D.D. Reynolds und B.C. Cossar (J. Heterocycl. Chem. 8 ,597 (1971)) hergestellt werden: Kp. 67-70°/1,8 mbar (Reinolds und Cossar: Kp. 92°/0,4 mm).

Beispiel 10: N-Allyl-N-tert-butylthiomethyl-(R)-glycidsäureamid (Ausgangsprodukt)

Analog Beispiel 5 wird aus 845 mg (5 mMol) 2-(S)-Brom-3-hydroxypropionsäure in Tetrahydrofuran mit 10 mMol 1,5-Diazabicyclo [5.4.0]undec-5-en in situ das DBU-Salz der (R)-Glycidsäure hergestellt, dieses in Methylenchlorid mit 1,03 g (5 mMol) Dicyclohexylcarbodiimid und mit 979 mg (5 mMol) Allyl-tert-butylthiomethylammoniumchlorid behandelt und das erhaltene Rohprodukt an Kieselgel in Gemischen aus Hexan und Ethylacetat (9:1) und (4:1) chromatographiert. Die Titelverbindung wird als farbloses Oel erhalten. Rf (Toluol: Ethylacetat 1:1): 0,40; IR ($CH_2Cl_2$): 3080-2850, 1660, 1458, 1440 (sh), 1410, 1360, 1300-1240, 1220 (sh), 1208, 1160, 980, 940, 910, 890.

Beispiel 11: N-Allyl-N-tert-butylsulfonylmethyl-(R)-glycidsäureamid (Ausgangsprodukt)

Zu einer Lösung von 573 mg (2,5 mMol) N-Allyl-N-tert-butylthiomethyl-(R)-glycidsäureamid in 20 ml Methylenchlorid wird bei -10° 1,05 g (ca. 5,5 mMol) 90 %ige m-Chlorperbenzoesäure in mehreren Portionen innerhalb von 15 Minuten gegeben und das Gemisch 2 Stunden bei 0° gerührt. Anschliessend wird mit Methylenchlorid verdünnt und nacheinander mit eiskalter 2 %iger wässriger Natriumbisulfit- und eiskalter 5 %iger wässriger Natriumbicarbonat-Lösung gewaschen. Die wässrigen Anteile werden mit Methylenchlorid nachextrahiert, die vereinigten $CH_2Cl_2$-Extrakte über Natriumsulfat getrocknet, eingedampft und der erhaltene ölige Rückstand an Kieselgel mit Toluol-Ethylacetat (4:1) chromatographiert. Die Titelverbindung wird als farbloses Oel erhalten. Rf (Toluol:Ethylacetat 4:1): 0,22; [α] = +44 ± 1° (1,55 % in $CHCl_3$).

Beispiel 12: 1-Allyl-3-(S)-hydroxymethyl-4-(R)-tert-butylsulfonyl-2-azetidinon

Eine Lösung von 197 mg (0,757mMol) N-Allyl-N-tert-butylsulfonylmethyl-(R)-glycidsäureamid in 1 ml Tetrahydrofuran wird bei 0° unter Feuchtigkeitsausschluss in 8 ml einer Tetra-n-butylammoniumfluorid/THF-Lösung eingetragen, welche aus 5,0 g Tetra-n-butylammoniumfluoridtrihydrat durch Entwässern bei 60°C/0,1 Torr und Auffüllen mit Tetrahydrofuran auf 20 ml hergestellt worden ist. Das Reaktionsgemisch wird 2 Stunden bei Raumtemperatur gerührt und anschliessend zwischen Methylenchlorid und Phosphatpuffer-Lösung (pH 8,0) verteilt. Das durch Trocknen über Natriumsulfat und Eindampfen der organischen Phase erhaltene Rohprodukt wird auf präparativen Kieselgel-Platten mit Hexan-Ethylacetat (1:3) chromatographiert. Die Titelverbindung wird in Form von farblosen Kristallen erhalten. F. 99 - 103°; Rf (Hexan-Ethylacetat 1:3): 0,18; [α] = 46 ± 1° (1,30 % in $CHCl_3$); $^1$H-NMR-Spektrum: δ = 4,96 für Proton (a) am 4-(R)-C-Atom und δ = 3,72 für Proton (b) am 3-(S)-C-Atom (trans-Verbindung), J a-b: ca. 2.

Beispiel 13: N-Allyl-N-phenylthiomethylammoniumchlorid (Ausgangsprodukt) wird nach der allgemeinen Vorschrift von D.D. Reynolds und B.C. Cossar, supra, folgendermassen hergestellt:

Eine Lösung von 20,73 g (0,1 Mol) 1,3,5-Triallylhexahydro-1,3,5-triazin in 150 ml Diethylether wird bei -20° zu einer Lösung von 12 g Chlorwasserstoff in 130 ml Diethylether getropft und die entstandene Suspension von weissen Kristallen 15 Minuten bei dieser Temperatur weitergerührt. Anschliessend werden 30,6 ml (0,3 Mol) Thiophenol in einer Portion zugegeben und das Reaktionsgemisch bei Raumtemperatur 20 Stunden gerührt. Die gebildeten Kristalle der Titelverbindung werden abgenutscht und am Filter mit Diethylether gewaschen. F. 100°.

Beispiel 14: N-Allyl-N-phenylthiomethyl-(R)-glycidsäureamid (Ausgangsprodukt)

Analog zu Beispiel 5 werden 8,45 g (50 mMol) 2-(S)-Brom-3-hydroxypropionsäure in 200 ml Tetrahydrofuran mit 15,2 g (100 mMol) 1,5-Diazabicyclo[5.4.0]undec-5-en in das DBU-Salz der (R)-Glycidsäure übergeführt. Dieses wird in situ in Methylenchlorid bei Raumtemperatur mit 10,31 g (50 mMol) Dicyclohexylcarbodiimid und anschliessend mit 10,75 g (50 mMol) N-Allyl-N-phenylthiomethylammoniumchlorid bei 0° versetzt. Das entstandene Gemisch wird 3 Stunden bei Raumtemperatur weitergerührt. Der kristalline Dicyclohexylharnstoff wird abgenutscht, das Filtrat mit Methylenchlorid versetzt und mit Phosphatpuffer, pH 8,0, gewaschen. Das aus der organischen Phase durch Trocknen über Natriumsulfat und Eindampfen im Vakuum erhaltene Rohprodukt wird auf 500 g Kieselgel mit Toluol-Ethylacetat (9:1) chromatographiert und die reine Titelverbindung als farbloses Oel erhalten. Rf (Toluol:Ethylacetat 1:1): 0,39; IR ($CH_2Cl_2$): 3050-2850, 1660, 1500, 1446, 1405 1360, 1340, 1201, 1080, 1020, 990, 907, 892.

Beispiel 15: N-Allyl-N-phenylsulfonylmethyl-(R)-glycidsäureamid (Ausgangsprodukt)

Eine Lösung von 1,99 g (8,52 mMol) N-Allyl-N-phenylthiomethyl-(R)-glycidsäureamid in 75 ml Methylenchlorid wird 2 Stunden bei 0° mit 3,59 g (ca. 18,7 mMol) 90%iger m-Chlorperbenzoesäure gerührt und das erhaltene Reaktionsgemisch nach Verdünnen mit Methylenchlorid mit 2 %iger, eiskalter, wässriger

Natriumbisulfit- und mit 5 %iger, eiskalter, wässriger Natriumbicarbonatlösung gewaschen. Das aus der organischen Phase durch Eindampfen im Vakuum erhaltene Rohprodukt wird aus Methylenchlorid-Diethylether kristallisiert und die reine Titelverbindung in Form weisser Kristalle erhalten. Ein weiterer Anteil an reiner Titelverbindung wird durch Chromatographieren des Mutterlaugen-Rückstands auf Kieselgel mit Toluol:Ethylacetat 9:1 erhalten. F. 82 - 84°; Rf (Toluol: Ethylacetat 1:1): 0,23; [α] = 22 ± 1° (1,02 % in $CHCl_3$). Das $^1H$-NMR-Spektrum (in $CDCl_3$, 400 MHz) zeigt eine Verdoppelung von Signalen und weist auf die Existenz von zwei Rotameren hin.

Beispiel 16: 1-Allyl-3(S)-hydroxymethyl-4-(R)-phenylsulfonyl-2-azetidinon

Eine Lösung von 201 mg (0,757 mMol) N-Allyl-N-phenylsulfonylmethyl-(R)-glycidsäureamid in 1 ml Tetrahydrofuran wird mit 8 ml einer Tetra-n-butylammoniumfluorid/THF-Lösung versetzt, welche aus 5 g Tetra-n-butylammoniumfluorid-trihydrat durch Trocknen bei 60°/0.1 Torr und Auffüllen mit Tetrahydrofuran auf 20,0 ml hergestellt worden ist. Das entstandene Reaktionsgemisch wird bei 0° 2 Stunden lang gerührt und anschliessend zwischen Methylenchlorid und Phosphatpuffer-Lösung, pH 8,0, verteilt. Das aus der organischen Schicht durch Eindampfen isolierte Rohprodukt wird auf präparativen Kieselgelplatten in Ethylacetat-Hexan (2:1) chromatographiert und die Titelverbindung als farbloses Oel erhalten. Rf (Hexan: Ethylacetat 1:2): 0,19; IR ($CH_2Cl_2$): 3600-3250 (mit Maximum bei 3590), 3050, 2910, 2850, 1763, 1440, 1375, 1320, 1305, 1250 (sh), 1145, 1082, 1032, 940; NMR-Spektrum: δ = 4,83 für Proton (a) am 4-(R)-C-Atom und δ = 3,52 für Proton (b) am 3-(S)-C-Atom (trans-Verbindung), J a-b: ca. 2.

Beispiel 17: 2,4-Dimethoxybenzyl-tert-butylthiomethylammoniumchlorid (Ausgangsprodukt)

Zu einer Lösung von 202 mg Chlorwasserstoff in 4 ml Acetonitril wird bei -25° eine Lösung von 895 mg (1,67 mMol) 1,3,5-Tris-(2,4-dimethoxybenzyl)-hexahydro-1,3,5-triazin in 10 ml Acetonitril zugetropft und das erhaltene Reaktionsgemisch weitere 15 Minuten bei dieser Temperatur gerührt. Nach Zugabe von 0,567 ml (5 mMol) tert-Butylmercaptan wird das Gemisch bei Raumtemperatur über Nacht weitergerührt. Nach Eindampfen unter vermindertem Druck, Aufnehmen mit Methylenchlorid und nochmaligem Eindampfen wird die rohe Titelverbindung als Schaum erhalten. Diese wird ohne weitere Reinigung in die nächste Stufe eingesetzt.

Das als Ausgangsmaterial verwendete 1,3,5-Tris-(2,4-dimethoxybenzyl)-hexahydro-1,3,5-triazin kann nach der allgemeinen Vorschrift von D.D. Reynolds und B.C. Cossar, supra, hergestellt werden; $^1H$-NMR-Spektrum (in $CDCl_3$ 60 MHz): 3,53 (S, 2H); 3,73 (S, 2H); 3,86 (breites S, 6H; 2 $OCH_3$); 6,36-6,64 (M, 2H); 7,20 - 7,50 (M, 1H).

Beispiel 18: N-2,4-Dimethoxybenzyl-N-tert-butylthiomethyl-(R)-glycidsäureamid (Ausgangsprodukt)

7,605 g (45 mMol) 2(S)-Brom-3-hydroxypropionsäure werden analog Beispiel 5 mit 13,7 g (90 mMol) 1,5-Diazabicyclo[5.4.0]undec-5-en in Tetrahydrofuran in das DBU-Salz der (R)-Glycidsäure überführt. Dieses wird in situ in Methylenchlorid bei Raumtemperatur mit 9,28 g (45 mMol) Dicyclohexylcarbodiimid und 45 mMol 2,4-Dimethoxybenzyl-tert-butylthiomethylammoniumchlorid versetzt. Der gebildete Dicyclohexylharnstoff wird abgenutscht und das Filtrat zwischen Methylenchlorid und Phosphatpuffer-Lösung, pH 8,0, verteilt. Nach Chromatographie des Rückstands und Eindampfen der organischen Phase auf einer 1000 g Kieselgel-Mitteldruckkolonne mit Toluol-Ethylacetat (9:1) erhält man die reine Titelverbindung als viskoses Oel. Rf (Toluol: Ethylacetat 1:1) 0,42; IR ($CH_2Cl_2$): 3050 (sh), 2930, 2800, 1660, 1605, 1580, 1500, 1450, 1360, 1287, 1250 (sh), 1205, 1158, 1126, 1030.

Beispiel 19: N-2,4-Dimethoxybenzyl-N-tert-butylsulfonylmethyl-(R)-glycidsäureamid (Ausgangsprodukt)

a) Eine Lösung von 492 g (14,43 mMol) N-2,4-Dimethoxybenzyl-N-tert-butylthiomethyl-(R)-glycidsäureamid in 120 ml Methylenchlorid wird bei -10° mit 6,08 g (ca. 31,75 mMol) 90 %iger m-Chlorperbenzoesäure versetzt und anschliessend bei 0° zwei Stunden gerührt. Das erhaltene Reaktionsgemisch wird nach Verdünnen mit Methylenchlorid nacheinander mit 2 %iger wässriger Natriumbisulfit- und 5 %iger wässriger Natriumbicarbonat-Lösung gewaschen. Der erhaltene Rückstand wird im Vakuum nach Eindampfen der organischen Phase auf 300 g Kieselgel in Toluol-Ethylacetat (4:1) chromatographiert und die Titelverbindung als farbloses, viskoses Oel erhalten. Rf (Toluol: Ethylacetat 1:1): 0,23; IR ($CH_2Cl_2$): 3050-2880, 1662, 1608, 1585, 1502, 1460, 1452 (sh), 1437, 1360, 1250 (sh), 1208, 1160, 1130, 1112, 1030. Das $^1$H-NMR-Spektrum (in $CDCl_3$, 360 MHz) weist auf die Existenz eines Hauptrotameren hin.

b) Die Titelverbindung lässt sich auch aus 2(S)-Brom-3-hydroxypropionsäure und 2,4-Dimethoxybenzyl-tert-butylthiomethylammoniumchlorid ohne Isolierung von N-2,4-Dimethoxybenzyl-N-tert-butylthiomethyl-(R)-glycidsäureamid in situ herstellen.

Beispiel 20: 1-(2,4-Dimethoxybenzyl)-3-(S)-hydroxymethyl-4-(R)-tert-butylsulfonyl-2-azetidinon

Zu einer Lösung von 3,35 g (9,0 mMol) N-2,4-dimethoxybenzyl-N-tert-butylsulfonylmethyl-(R)-glycidsäureamid in 14 ml Tetrahydrofuran werden bei 0° unter Feuchtigkeitsausschluss 53 ml einer Lösung von Tetra-n-butylammoniumfluorid in THF zugegeben, die man aus 20 g Tetra-n-butylammoniumfluorid-trihydrat (Fluka), Entwässern bei 60°/0,1 Torr und Auffüllen auf 80 ml mit Tetrahydrofuran hergestellt hat. Das Reaktionsgemisch wird mit 50 g frisch bei 0,1 Torr aktiviertem Molekularsieb (Typ 4A 1/16, Fa. Dr. Bender & Dr. Hobein) versetzt und 2 Stunden bei 0° gerührt. Das Molekularsieb wird abfiltriert, mit 360 ml Methylenchlorid gewaschen, und die vereinigten Filtrate nach Versetzen mit 1,8 l Diethylether mit 200 ml einer Phosphatpufferlösung, pH 8,0, gewaschen. Nach Abdestillieren der organischen Lösungsmittel wird der Rückstand an 110 g Kieselgel mit Toluol-Ethylacetat (2:1) und (1:1) chromatographiert. Man erhält die Titelverbindung, die nach Umkristallisieren aus Methylenchlorid-Ether-Pentan weisse Kristalle bildet. F. 124-125°; [α] = +10 ± 1° (1,09 % in $CHCl_3$); $^1$H-NMR-Spektrum: δ = 4,61 für Proton (a) am 4-(R)-C-Atom und δ = 3,69 für Proton (b) am 3-(S)-C-Atom (trans-Verbindung), J a-b: ca. 2.

Beispiel 21: 1-(2,4-Dimethoxybenzyl)-3-(S)-dimethyl-tert-butylsilyloxymethyl-4-(R)-tert-butylsulfonyl-2-azetidinon

Eine Lösung von 870 mg (2,34 mMol) 1-(2,4-Dimethoxybenzyl)-3-(S)-hydroxymethyl-4-(R)-tert-butylsulfonyl-2-azetidinon, 706 mg (4,68 mMol) tert-Butyldimethylchlorsilan und 318 mg (4,68 mMol) Imidazol in 8 ml Dimethylformamid wird 1,5 Stunden bei Raumtemperatur gerührt und im Hochvakuum bei 25° eingedampft. Der Rückstand wird in Ethylacetat aufgenommen und nacheinander mit Wasser, 8 %iger wässriger $NaHCO_3$-Lösung, und nochmals mit Wasser gewaschen, wobei alle wässrigen Anteile mit Ethylacetat nachextrahiert werden. Die vereinigten organischen Phasen werden über $Na_2SO_4$ getrocknet unter vermindertem Druck eingedampft und der erhaltene Rückstand an 30 g Merck Kieselgel mit Toluol: Ethylacetat (4:1) chromatographiert. Man erhält die kristaline Titelverbindung. F. 113-114° (aus $CH_2Cl_2$-$Et_2O$-Pentan); Rf (Ethylacetat:Hexan 3:1): 0,61; [α] = -6 ± 1° (1,10 % in $CHCl_3$).

Beispiel 22: 3-(S)-Dimethyl-tert-butylsilyloxymethyl-4-(R)-tert-butylsulfonyl-2-azetidinon

a) Eine Lösung von 97 mg (02 mMol) 1-(2,4-Dimethoxybenzyl)-3-(S)-dimethyl-tert-butylsilyloxymethyl-4-(R)-tert-butylsulfonyl-2-azetidinon, 486 mg (1,8 mMol) Kaliumperoxodisulfat und 174 mg (1,0 mMol) Dikaliumhydrogenphosphat in 4 ml Wasser und 4 ml Acetonitril wird 3,5 Stunden auf 65° erhitzt. Das Lösungsmittel wird unter vermindertem Druck abdestilliert, und der wässrige Rückstand mit Ethylacetat extrahiert. Das erhaltene Rohprodukt wird auf präparativen Kieselgel-Platten mit Toluol-Ethylacetat (4:1) chromato graphiert und die Titelverbindung erhalten. F. 141-145° (aus $CH_2Cl_2$-$Et_2O$-Pentan); Rf (Toluol:Ethylacetat 4:1): 0,18; [α] = +27 $CH_2Cl_2$-$Et_2O$-Pentan); Rf (Toluol:Ethylacetat 4:1): 0,18; [α] = +27 ± 1° (0,975 % in $CHCl_3$).

Die Titelverbindung kann auch wie folgt erhalten werden:

b) Eine Lösung von 17 mg 3-(S)-Hydroxymethyl-4-(R)-tert-butylsulfonyl-2-azetidinon (Beispiel 23), 14,6 mg Imidazol und 31 mg Dimethyl-tert-butylchlorsilan in 0,4 ml Dimethylformamid wird 75 Minuten bei Raumtemperatur gerührt, das Lösungsmittel im Hochvakuum bei 25° abgedampft, der Rückstand in Ethylacetat aufgenommen und mit drei kleinen Portionen Wasser gewaschen. Nach Trocknen der organischen Phase über Natriumsulfat und Abdestillieren des Lösungsmittels erhält man die kristalline Titelverbindung.

Beispiel 23: 3-(S)-Hydroxymethyl-4-(R)-tert-butylsulfonyl-2-azetidinon

Eine Lösung von 74 mg (0,2 mMol) 1-(2,4-Dimethoxybenzyl)-3-(S)-hydroxymethyl-4-(R)-tert-butylsulfonyl-2-azetidinon (siehe Beispiel 20), 486 mg (1,8 mMol) Kaliumperoxodisulfat und 174 mg (1,0 mMol) Dikaliumhydrogenphosphat in 4 ml Acetonitril wird 2 Stunden bei 65° unter Rückflusskühler gerührt, das erhaltene Reaktionsgemisch unter vermindertem Druck von Acetonitril befreit und der wässrige Rückstand mit Ethylacetat extrahiert. Nach Trocknen der organischen Phase über $Na_2SO_4$ und Eindampfen im Vakuum erhält man einen Rückstand, aus dem durch Versetzen mit etwas Methylenchlorid und Ether die Titelverbindung in Form farbloser Kristalle erhalten wird, F. 196°; Rf (Ethylacetat): 0,20; [α] = +25 ± 1° (0,815 % in $CH_3OH$).

Beispiel 24: N-p-Methoxybenzyl-N-tert-butylthiomethylammoniumchlorid (Ausgangsprodukt)

Eine Lösung von 10,69 g (23,9 mMol) 1,3,5-Tris-(p-methoxybenzyl)-hexahydro-1,3,5-triazin, welches analog den Vorschriften in der Deutschen Offenlegungsschrift DE-A-2,431,862 hergestellt werden kann, in 170 ml Acetonitril wird bei Raumtemperatur nacheinander mit einer Lösung von 2,88 g (78,8 mMol) Chlorwasserstoff in 20 ml Acetonitril und 6,45 g (71,66 mMol) tert-Butylmercaptan versetzt.

Das Gemisch wird 22 Stunden gerührt. Man nutscht ungelöstes Material ab und engt das Filtrat unter vermindertem Druck ein. Man erhält einen kristallinen Rückstand, der mit Ether verrührt und abgesaugt wird. F. 142°.

Beispiel 25: N-p-Methoxybenzyl-N-tert-butylthiomethyl-(R)-glycidsäureamid (Ausgangsprodukt)

Aus 845 mg (5 mMol) 2-(S)-Brom-3-hydroxypropionsäure wird in 15 ml Tetrahydrofuran mit 10 mMol 1,5-Diazabicyclo[5.4.0]undec-5-en analog Beispiel 5 das DBU-Salz der (R)-Glycidsäure hergestellt. Dieses wird in 20 ml Methylenchlorid nacheinander mit 1,03 g (5 mMol) Dicyclohexylcarbodiimid mit 1,38 g ( 5 mMol) N-p-Methoxybenzyl-N-tertbutylthiomethylammoniumchlorid bei Raumtemperatur versetzt und 2,5 Stunden gerührt. Der ausgeschiedene Dicyclohexylharnstoff wird abfiltriert, das Filtrat mit Methylenchlorid verdünnt und mit wässrigem Phosphatpuffer (pH 8) gewaschen. Nach Trocknen der organischen Phase über Natriumsulfat und Eindampfen unter vermindertem Druck chromatographiert man den Rückstand an 70 g Kieselgel mit Toluol-Ethylacetat (4:1). Man erhält die Titelverbindung als farbloses Oel. Rf (Toluol-Ethylacetat 1:1): 0,60; IR (in Methylenchlorid): 3000-2850, 1693, 1660, 1610, 1580, 1508, 1460-1450, 1415-(sh), 1364, 1242, 1210, 1180, 1110, 1037.

Beispiel 26: N-p-Methoxybenzyl-N-tert-butylsulfonylmethyl-(R)-glycidsäureamid (Ausgangsprodukt)

2,0 g rohes N-p-Methoxybenzyl-N-tert-butylthiomethyl-(R)-glycidsäureamid wird in 50 ml Methylenchlorid bei -10° mit 2,11 g 90 %iger m-Chloperbenzoesäure versetzt. Das Gemisch wird 45 Minuten lang gerührt und nach Zugabe von weiteren 480 mg 90 %iger m-Chlorperbenzoesäure die Oxydationsreaktion 45 Minuten fortgesetzt. Man nutscht anschliessend die ausgeschiedene m-Chlorbenzoesäure ab, verdünnt das Filtrat mit Methylenchlorid und wäscht mit 3 %iger wässriger Natriumbisulfit- und 8 %iger wässriger Natriumbicarbonatlösung. Die organische Phase wird über Natriumsulfat getrocknet und unter vermindertem Druck eingedampft. Man chromatographiert den Rückstand an 75 g Kieselgel mit Toluol-Ethylacetat (4:1) und erhält die Titelverbindung als dickflüssiges, farbloses Oel. Rf (Toluol: Ethylacetat 1:1): 0,40; IR (in Methylenchlorid): 3000-2850, 1675, 1610, 1580, 1512, 1460, 1438, 1397, 1363, 1302, 1240, 1180, 1120, 1038.

Beispiel 27: 1-p-Methoxybenzyl-3-(S)-hydroxymethyl-4-(R)-tert-butylsulfonyl-2-azetidinon

Analog Beispiel 8b wird aus 1,71 g (5 mMol) N-p-Methoxybenzyl-N-tert-butylsulfonylmethyl-(R)-glycidsäureamid in THF durch Reaktion mit entwässertem Tetra-n-butylammoniumfluorid in Gegenwart von aktiviertem Molekularsieb die Titelverbindung als Rohprodukt erhalten, welche auf Kieselgel mit Toluol-Ethylacetat (4:1) und (2:1) chromatographiert wird. Man erhält die reine Titelverbindung. F. 121-122° (aus Methylenchlorid-Ether); [α] = +4 ± 1° (0,104 % in Chloroform).

Beispiel 28: 3-(S)-Hydroxymethyl-4-(R)-tert-butylsulfonyl-2-azetidinon

Eine Lösung von 68,3 mg (0,2 mMol) 1-p-Methoxybenzyl-3-(S)-hydroxymethyl-4-(R)-tert-butylsulfonyl-2-azetidinon in 4 ml Acetonitril wird zu einer Lösung von 428 mg (1,8 mMol) Natriumperoxodisulfat, 174 mg (1 mMol) Dikaliumhydrogenphosphat, 1 mg Eisen-(II)-sulfatheptahydrat und 2 mg Kupfer(II)-acetathydrat in 4 ml Wasser gegeben. Das Gemisch wird 2 Stunden unter Argon-Atmosphäre bei 65° Badtemperatur gerührt. Danach wird das erhaltene Gemisch unter vermindertem Druck auf das halbe Volumen eingeengt und mit Ethylacetat extrahiert. Der Extrakt wird mit Methylenchlorid und Ether verrieben und die reine Titelverbindung erhalten.

Beispiel 29: p-Methoxybenzyl-3-(S)-dimethyl-tert-butylsilyloxymethyl-4-(R)-tert-butylsulfonyl-2-azetidinon

5,46 mMol 1-p-Methoxybenzyl-3-(S)-hydroxymethyl-4-(R)-tert-butylsulfonyl-2-azetidinon wird als Rohprodukt in 20 ml Dimethyl formamid mit 1,65 g (2 Aequiv.) Dimethyl-tert-butylchlorsilan und 0,74 g Imidazol 90 Minuten bei Raumtemperatur gerührt. Man destilliert das Lösungsmittel im Hochvakuum ab und wäscht den in Ethyl acetat gelösten Rückstand mit wässriger Natriumbicarbonat-Lösung. Nach Eindampfen der über Natriumsulfat getrockneten organischen Phase unter vermindertem Druck und Chromatographieren des Rohprodukts an 60 g Kieselgel mit Toluol-Ethylacetat (4:1) erhält man die reine Titelverbindung. F. 104-105°; Rf (Hexan: Ethylacetat 1:3): 0,78; [α] = + 20 ± 1°; $^1$H-NMR-Spektrum: δ = 4,69 für Proton (a) am 4(R)-C-Atom und δ = 3,70 für Proton (b) am 3-(S)-C-Atom (trans-Verbindung) J a-b: ca. 2.

Beispiel 30: N-p-Methoxybenzyl-N-tert-butylthiomethyl-2-(S)-brom-3-hydroxypropionsäureamid - (Ausgangsprodukt)

Zu einem Gemisch von 3,38 g (20 mMol) 2-(S)-Brom-3-hydroxypropionsäure und 5,151 g (20 mMol) p-Methoxybenzyl-tert-butylthiomethyl ammoniumchlorid (Beispiel 24) in 100 ml Tetrahydrofuran werden unter Rühren bei Raumtemperatur 4,13 g (20 mMol) Dicyclohexylcarbodiimid und 2,79 ml (20 mMol) Triäthylamin gegeben. Man rührt das Gemisch zwei Stunden lang bei Raumtemperatur und nutscht den ausgeschiedenen Dicyclohexylharnstoff ab. Das Filtrat wird bei vermindertem Druck eingedampft und der Rückstand an 500 g Kieselgel mit Toluol-Ethylacetat (9:1) chromatographiert. Man erhält die Titelverbindung als farbloses, dickflüssiges Oel. Rf (Toluol: Ethylacetat 1:1): 0,43; IR (in Methylenchlorid): 3550-3250, 2970-2850, 1640, 1610, 1580, 1508, 1460-1430, 1410 (sh), 1362, 1241, 1175, 1148, 1109, 1034; Das $^1$H-NMR-Spektrum weist auf die Existenz von zwei Rotameren hin.

Beispiel 31: N-p-Methoxybenzyl-N-tert-butylsulfonylmethyl-2(S)-brom-3-hydroxypropionsäureamid - (Ausgangsprodukt)

Eine Lösung von 3,90 g (10 mMol) N-p-Methoxybenzyl-N-tert-butylthiomethyl-2-(S)-brom-3-hydroxypropionsäureamid in 100 ml Methylen chlorid wird bei -10° portionsweise mit 4,21 g (22 mMol) 90 %iger m-Chlorperbenzoesäure versetzt und 75 Minuten bei 0° gerührt. Das Reaktionsgemisch wird mit 100 ml Methylenchlorid verdünnt und mit 100 ml 3 %iger wässriger Natriumbisulfit- und 100 ml 8 %iger wässriger Natriumbicarbonat-Lösung gewaschen und die wässrigen Anteile mit Methylenchlorid nachextrahiert. Nach Trocknen der organischen Phase über Natriumsulfat und Eindampfen unter ver mindertem Druck erhält man das Rohprodukt. Nach Chromatographieren an 200 g Kieselgel mit Toluol-Ethylacetat (4:1) erhält man die Titelverbindung als farbloses, dickflüssiges Oel. Rf (Toluol: Ethylacetat 1:1): 0,36; [α] = +93 ± 1° (1,02 % in Chloroform); Das $^1$H-NMR-Spektrum weist auf die Existenz von zwei Rotameren hin.

Beispiel 32: 1-p-Methoxybenzyl-3-(S)-hydroxymethyl-4-(R)-tert-butylsulfonyl-2-azetidinon

Eine Lösung von 1,426 g (3,38 mMol) N-p-Methoxybenzyl-N-tert-butylsulfonylmethyl-2-(S)-brom-3-hydroxypropionsäureamid in 6 ml Tetrahydrofuran wird bei 0° tropfenweise mit einer Lösung von entwässertem Tetra-n-butylammoniumfluorid in Tetrahydrofuran versetzt, welche man aus Tetra-n-butylammoniumfluorid-trihydrat durch Trocknen bei 55-60°/0,1 mm und Auflösen in 40 ml Tetrahydrofuran herstellt. Das Reaktionsgemisch wird mit aktiviertem Molekularsieb (4 Å) versetzt und 2 Stunden bei 0°

gerührt. Man filtriert das Molekularsieb ab, wäscht dieses mit Methylenchlorid nach, versetzt die vereinigten Filtrate mit 600 ml Ether und 75 ml Pufferlösung vom pH 8. Nach Trocknen über Natriumsulfat und Eindampfen im Vakuum erhält man das Rohprodukt, welches man chromatographisch an 50 g Kieselgel mit Toluol:Ethylacetat (4:1) und (2:1) reinigt.

Beispiel 33: N-p-Methoxybenzyl-N-tert-butylthiomethyl-2-(S)-brom-3-(R)-hydroxybutyramid - (Ausgangsprodukt)

Eine Lösung von 1,83 g (10 mMol) 2-(S)-Brom-3-(R)-hydroxybuttersäure, hergestellt analog nach einer Vorschrift von Shimohigashi Y. et al., Bull. Chem. Soc. Japan 52 , 949 (1979), wird bei Raumtemperatur nacheinander mit 2,76 g (10 mMol) p-Methoxybenzyl-tert-butylthiomethylammoniumchlorid (Beispiel 24), 2,06 g (10 mMol) Dicyclohexylcarbodiimid und tropfenweise mit 1,40 ml (10 mMol) Triethylamin versetzt. Das erhaltene Reaktionsgemisch wird bei Raumtemperatur zwei Stunden lang gerührt. Man nutscht den ausgeschiedenen Dicyclohexylharnstoff ab, verdünnt das Filtrat mit Methylenchlorid und wäscht mit Wasser und Phosphatpufferlösung vom pH 8. Man trocknet die organische Phase über Natriumsulfat, dampft ein und chromatographiert den öligen Rückstand an Kieselgel mit Toluol-Ethylacetat. Man erhält die Titelverbindung als farbloses, dickflüssiges Oel. Rf (Toluol-Ethylacetat 1:1): 0,55; IR (in Methylenchlorid): 3550-3200, 2950-2850, 1632, 1608, 1508, 1457, 1438, 1407, 1360, 1242, 1202, 1175, 1150, 1028.

Beispiel 34: N-p-Methoxybenzyl-N-tert-butylsulfonylmethyl-2-(S)-brom-3-(R)-hydroxybutyramid - (Ausgangsprodukt)

Eine Lösung vo 1,97 g (4,89 mMol) N-p-Methoxybenzyl-N-tert-butylthiomethyl-2-(S)-brom-3-(R)-hydroxybutyramid in 50 ml Methylen chlorid wird bei -14° unter Rühren mit 2,06 g (ca. 2,2 Aequivalente) 90 %iger m-Chlorperbenzoesäure versetzt. Das Reaktionsgemisch wird 80 Minuten bei 0° gerührt. Man filtriert die ausgeschiedene m-Chlorbenzoesäure ab, verdünnt das Filtrat mit Methylenchlorid und schüttelt dieses nacheinander mit 3 %iger wässriger Natriumhydrogensulfit-und 8 %iger wässriger Natriumhydrogencarbonat-Lösung. Man trocknet die organische Phase über Natriumsulfat, dampft unter vermindertem Druck ein und chromatographiert den Rückstand an Kieselgel mit Toluol-Ethylacetat (7:1) und (6:1). Man erhält die Titelverbindung als farbloses, dickflüssiges Oel. Rf (Toluol-Ethylacetat 1:1): 0,43; $[\alpha]$ = +88 $\pm$ 1° (1,01 % in Chloroform). Das $^1$H-NMR-Spektrum (400 MHz in $CDCl_3$) weist auf die Existenz von zwei Rotameren im Verhältnis von 1,3:1 hin.

Beispiel 35: N-p-Methoxybenzyl-N-tert-butylthiomethyl-(2R,3R)-2,3-epoxybutyramid (Ausgangsprodukt)

Eine Lösung von 1,83 g (10 mMol) 2-(S)-Brom-3-(R)-hydroxybuttersäure und 3,045 g (20 mMol) 1,5-Diazabicyclo[5.4.0]undec-5-en in 30 ml Tetrahydrofuran wird eine Stunde bei Raumtemperatur gerührt und unter vermindertem Druck eingeengt. Der Rückstand wird in 40 ml Methylenchlorid gelöst. Nach Zugabe von 2,06 g (10 mMol) Dicyclohexylcarbodiimid und 2,76 g (10 mMol) p-Methoxybenzyl-tert-butylthiomethylammoniumchlorid wird 2,5 Stunden bei Raumtemperatur gerührt. Man filtriert den gebildeten Dicyclohexylharnstoff ab, verdünnt das Filtrat mit Methylenchlorid und wäscht mit wässriger Phosphatpufferlösung vom pH 8. Man trocknet die organische Phase über Natriumsulfat, dampft im Vakuum ein und chromatographiert den Rückstand an Kieselgel mit Toluol-Ethylacetat (9:1). Man erhält die Titelverbindung als farbloses, dickflüssiges Oel. Rf (Toluol-Ethylacetat 1:1): 0,40. IR (in Methylenchlorid): 3050-2850, 1690, 1660, 1640 (sh), 1607, 1506, 1460-1438, 1400 (sh), 1362, 1240, 1204, 1175, 1150, 1133, 1108, 1035.

Beispiel 36: N-p-Methoxybenzyl-N-tert-butylsulfonylmethyl-(2R,3R)-2,3-epoxybutyramid (Ausgangsprodukt)

a) Zu einer Lösung von 486 mg (1,1 mMol) N-p-Methoxybenzyl-N-tert-butylsulfonylmethyl-2-(S)-brom-3-(R)-hydroxybutyramid (Beispiel 34) in 8 ml Tetrahydrofuran werden bei -14° und Feuchtigkeitsausschluss 340 ml 1,5-Diazabicyclo[5.4.0]undec-5-en in 1 ml Tetrahydrofuran gegeben. Die Lösung wird 75 Minuten bei Raumtemperatur gerührt. Nach Zugabe von Methylenchlorid wird die organische Phase mit 15 %iger wässriger Zitronensäure-Lösung und 8 %iger wässriger Natriumhydrogencarbonat-Lösung ausgeschüttelt. Die organische Phase wird über Natriumsulfat getrocknet und unter vermindertem Druck

eingedampft. Nach Chromatographieren des Rückstandes an Kieselgel mit Toluol-Ethylacetat (4:1) erhält man die Titelverbindung als farbloses, dickflüssiges Oel. Rf (Toluol-Ethylacetat 1:1): 0,29; [α] = + 45 ± 1° (1,065 % in $CHCl_3$). Das $^1$H-NMR-Spektrum (400 MHz in $CDCl_3$) weist auf die Existenz von zwei Rotameren im Verhältnis 1:2,8 hin.

b) 1,80 g (5,57 mMol) N-p-Methoxybenzyl-N-tert-butylthiomethyl-(2R,3R)-2,3-epoxybutyramid werden in 50 ml Methylenchlorid bei 0° mit 2,35 g (ca. 12,25 mMol) m-Chlorperbenzoesäure neunzig Minuten lang gerührt. Man filtriert die ausgeschiedene m-Chlorbenzoesäure ab, verdünnt das Filtrat mit Methylenchlorid und schüttelt dieses nacheinander mit 3 %iger wässriger Natriumhydrogensulfit- und 8 %iger wässriger Natriumhydrogencarbonatlösung aus. Man trocknet die organische Phase über Natriumsulfat, dampft unter vermindertem Druck ein und chromatographiert den Rückstand an Kieselgel mit Toluol-Ethylacetat (9:1) und (4:1). Man erhält die Titelverbindung.

Beispiel 37: 1-p-Methoxybenzyl-3-(S)-(1'-(R)-hydroxyethyl)-4-(R)-tertbutylsulfonyl-2-azetidinon

a) Eine Lösung von 398 mg (1,12 mMol) N-p-Methoxybenzyl-N-tert-butylsulfonylmethyl-(2R,3R)-2,3-epoxybutyramid in 2,5 ml Tetra hydrofuran wird bei 0° unter Rühren und Feuchtigkeitsausschluss tropfenweise mit 7 ml einer Lösung von entwässertem Tetra-n-butylammoniumfluorid in THF, hergestellt durch Entwässern von 5 g Tetra-n-butylammoniumfluorid-Trihydrat bei 55° und 0,1 Torr und Auffüllen auf 20 ml Tetrahydrofuran, versetzt. Man versetzt das Reaktionsgemisch mit aktiviertem Molekularsieb von 4 Å und rührt zwei Stunden. Man nutscht das Molekularsieb ab und wäscht dieses viermal mit je 20 ml Methylenchlorid nach. Jedes Filtrat wird separat mit 5 Teilen Diethylether versetzt und nacheinander mit wässriger Phosphatpufferlösung vom pH 8 gewaschen. Man tocknet die vereinigten organischen Phasen über Magnesiumsulfat und dampft unter vermindertem Druck ein. Man chromatographiert den Rückstand an 20 g Kieselgel mit Toluol-Ethylacetat (3:1) und erhält die kristalline Titelverbindung. F. 112°-113° (Kofler, aus Methylenchlorid, Diethylether, Pentan); Rf (Toluol-Ethylacetat 1:1): 0,27; [α] = +9 + 1° (1,105 % in Chloroform); $^1$H-NMR-Spektrum (400 Mhz in $CDCl_3$): δ = 4,65 für Proton (a) am 4(R)-C-Atom, δ = 3,61 für Proton (b) am 3(S)-C-Atom und δ = 4,09 für Proton (c) am 1'(R)-C-Atom der Hydroxyethylgruppe; J a-b: ca. 2, J b-c: ca. 7.

b) Analog Beispiel 37a) wird aus 803 mg (1,84 mMol) N-p-Methoxybenzyl-N-tert-butylsulfonylmethyl-2-(S)-brom-3-(R)-hydroxybutyramid (Beispiel 34) in 5 ml Tetrahydrofuran durch Zugabe von entwässertem Tetrabutylammoniumfluorid in Tetrahydrofuran in Gegenwart von aktiviertem Molekularsieb nach zwei Stunden Reaktionszeit bei 0° und Aufarbeitung die Titelverbindung erhalten.

Beispiel 38: 3-(S)-(1'-(R)-hydroxyethyl)-4-R-tert-butylsulfonyl-2-azetidinon

a) Eine Lösung von 71 mg (0,2 mMol) 1-p-Methoxybenzyl-(3)-S-(1'-(R)-hydroxyethyl)-4(R)-tert-butylsulfonyl-2-azetidinon in 4 ml Aceto nitril wird zu einer auf 65° erwärmten, gut gerührten Lösung von 428 mg (1,8 mMol) Natriumperoxodisulfat, 174 mg (1 mMol) Dikaliumhydrogenphosphat, 1 mg Eisen(II)-sulfatheptahydrat und 2 mg Kupfer(II)-acetathydrat in 4 ml Wasser gegeben. Man rührt das Reaktionsgemisch zwei Stunden lang bei 65°. Man destilliert das organische Lösungsmittel unter vermindertem Druck ab und extrahiert den wässrigen Rückstand mit Ethylacetat. Nach Einengen der organischen Phase im Vakuum kristallisiert man den Rückstand aus Methylenchlorid-Diethylether und erhält die Titelverbindung. F. 194°; Rf (Ethylacetat): [α] = +13 + 1° (0,75 % in Methanol); $^1$H-NMR-Spektrum (400 MHz in $CD_3OD$): δ = 5,04 für Proton (a) am 4(R)-C-Atom, δ = 3,58 für Proton (b) am 3(S)-C-Atom und δ = 4,18 für Proton (c) am 1'(R)-C-Atom der Hydroxyethylgruppe; J a-b; ca. 2, J b-c: ca. 4,5.

b) Zu einer Lösung von 710 mg (2 mMol) 1-p-Methoxybenzyl-3-(S)-(1'-(R)-hydroxyethyl)-4(R)-butylsulfonyl-2-azetidinon in 20 ml Acetonitril wird unter Rühren bei 0° eine Lösung von 4,172 g (7,6 mMol) Cer(IV)-ammoniumnitrat in 10 ml Wasser zugetropft und das Gemisch eine Stunde bei Raumtemperatur gerührt. Das Gemisch wird mit 50 ml Ethylacetat verdünnt und die abgetrennte wässrige Schicht zweimal mit 50 ml Ethylacetat extrahiert. Die vereinigten organischen Phasen werden über Natriumsulfat getrocknet und unter vermindertem Druck eingedampft. Der Rückstand wird mit etwas Methanol versetzt und mit Diethylether verrührt, so dass der oxidativ entstandene p-Methoxybenzaldehyd sich löst und man die Titelverbindung in Form von weissen Kristallen erhält, welche man anschliessend abnutscht, mit Diethylether wäscht und die mit der gemäss Beispiel 38 a) erhältlichen Verbindung identisch ist.

Beispiel 39: 3-(S)-(1'-(R)-tert-Butyldimethylsilyloxyethyl)-4-(R)-tert-butylsulfonyl-2-azetidinon

135 mg (0,574 mMol) 3-(S)-(1'-(R)-hydroxyethyl-(R)-tert-butylsulfonyl-2-azetidinon werden mit 173 mg

(1,15 mMol) tert-Butyldi methylchlorsilan und 78 mg (1,15 mMol) Imidazol in 3 ml Dimethylformamid zwei Stunden lang bei Raumtemperatur gerührt. Das Reaktionsgemisch wird im Vakuum eingedampft. Der Rückstand wird in Ethylacetat aufgenommen und mit 8 %iger wässriger Natriumhydrogencarbonat-Lösung gewaschen. Nach Trocknen der organischen Phase über Natriumsulfat und Eindampfen unter vermindertem Druck erhält man die Titelverbindung als kristallinen Rückstand. Durch Chromatographieren an Kieselgel mit Toluol-Ethylacetat (4:1) befreit man diesen von Spuren Imidazol und erhält weisse Kristalle. F. 196°; Rf (Toluol-Ethylacetat 1:1): 0,48; [α] = +11 ± 1°.

Beispiel 40: 1-p-Methoxybenzyl-3(S)-(1'-(R)-trichlorethoxycarbonyloxyethyl)-4-(R)-tert-butylsulfonyl-2-azetidinon

Eine Lösung von 1,777 g (5 mMol) 1-p-Methoxybenzyl-3-(S)-(1'-(R)-hydroxyethyl)-4-(R)-tert-butylsulfonyl-2-azetidinon (Beispiel 37), 0,9 ml (6,5 mMol) Triethylamin, 0,883 ml (6,5 mMol) Chlorameisensäure-2,2,2-trichlorethylester und 643 mg (5,25 mMol) 4-Dimethylaminopyridin in 25 ml Methylenchlorid wird 16 Stunden bei 5° gerührt, anschliessend mit 100 ml Methylenchlorid verdünnt und mit 50 ml 5 %-iger wässriger Zitronensäurelösung und 50 ml 8 %iger wässriger Natriumhydrogencarbonatlösung gewaschen. Die wässrige Phase wird mit Methylenchlorid extrahiert und die vereinigten organischen Extrakte über Natriumsulfat getrocknet und im Vakuum eingedampft. Das Rohprodukt wird auf 100 g Merck-Kieselgel mit Toluol-Ethylacetat (9:1) chromatographiert und die Titelverbindung als farblose Kristalle erhalten. F. 142-144°; Rf (Toluol-Ethylacetat 4:1): 0,49; [α] = + 36° ± 1° (0,958 % in Chloroform).

Beispiel 41: 3-(S)-(1'-(R)-Trichlorethoxycarbonyloxyethyl-4-(R)-tert-butylsulfonyl-2-azetidinon

Eine Lösung von 1,062 g (2 mMol) 1-p-Methoxybenzyl-3(S)-(1'-(R)-trichlorethoxycarbonyloxyethyl)-4-(R)-tert-butylsulfonyl-2-azetidinon in 20 ml Acetonitril wird bei 5° mit einer Lösung von 4,17 g (7,6 mMol) Cer(IV)-ammoniumnitrat in 10 ml Wasser versetzt und bei Raumtemperatur eine Stunde lang gerührt. Man extrahiert das Gemisch zweimal mit 75 ml Ethylacetat, wäscht die organischen Phasen mit 75 ml 8 %iger wässriger Natriumhydrogencarbonat-Lösung, trocknet diese über Natriumsulfat und dampft diese unter vermindertem Druck ein. Das Rohprodukt wird auf 50 g Merck-Kieselgel mit Toluol-Ethylacetat (4:1) chromatographiert und die Titelverbindung kristallin erhalten. F. 165-166°; Rf (Toluol-Ethylacetat 1:1): 0,52; [α] = + 38° ± 1° (1,499 % in Chloroform).

Beispiel 42: 1-p-Methoxybenzyl-3-(S)-(1'-(R)-allyloxycarbonyloxyethyl)-4(R)-tert-butylsulfonyl-2-azetidinon

Ein Zweiphasensystem bestehend aus einer Lösung von 1,77 g (5 mMol) 1-p-Methoxybenzyl-3-(S)-(1'-(R)-hydroxyethyl)-4-(R)-tert-butylsulfonyl-2-azetidinon (Beispiel 37) in 20 ml Methylenchlorid und aus 20 ml einer 1 N wässrigen Natriumhydroxid-Lösung wird mit 0,68 g (2 mMol) Tetra-n-butylammoniumhydrogensulfat versetzt. Unter starkem Rühren werden bei 0° 0,8 ml (7,5 mMol) Chlorameisensäureallylester hinzugegeben. Nach 20 und 40 Minuten setzt man nochmals 0,8 ml Chlorameisensäureallylester hinzu. Nach 60 Minuten Reaktionszeit wird das Gemisch mit Methylenchlorid versetzt, die wässrige Phase abgetrennt und die organische Phase nacheinander mit 5 %-iger wässriger Citronensäurelösung und mit 8 %-iger wässriger Natriumhydrogencarbonatlösung gewaschen. Nach Trocknen der organischen Phase über Natriumsulfat und Eindampfen unter vermindertem Druck erhält man das Rohprodukt als Rückstand, welches man chromatographisch an Merck-Kieselgel mit Toluol-Essigester (9:1) reinigt. F. 90 - 91°; Rf (Toluol-Ethylacetat 4:1): 0,43; [α] = + 46° ± 1° (1,49 % in Chloroform).

Beispiel 43: 3-(S)-(1'(R)-Allyloxycarbonyloxyethyl)-4-(R)-tert-butylsulfonyl-2-azetidinon

Eine Lösung von 518 mg (1,18 mMol) 1-p-Methoxybenzyl-3(S)-(1'-(R)-allyloxycarbonyloxyethyl)-4-(R)-tert-butylsulfonyl-2-azetidinon in 12 ml Acetonitril wird bei 0° mit einer Lösung von 2,46 g (4,48 mMol) Cer-(IV)-ammoniumnitrat in 6 ml Wasser versetzt und eine Stunde bei Raumtemperatur gerührt. Nach Extraktion mit Ethylacetat, Trocknen der organischen Phase über Natriumsulfat und Eindampfen unter vermindertem Druck erhält man das Rohprodukt, welches man chromatographisch an 20 g Merck-Kieselgel mit Toluol-Ethylacetat (4:1 und 1:1) reinigt. F. 137 - 138°; [α] = + 49 ± 1° (1,067 % in Chloroform); Rf (Toluol-Ethylacetat 1:1): 0,48.

Beispiel 44: (2S,3R)-2-Brom-3-hydroxybuttersäure-p-methoxybenzylamid (Ausgangsprodukt)

Zu einer bei Raumtemperatur unter Argonatmosphäre gerührten Lösung von 5 g (2S,3R)-2-Brom-3-hydroxybuttersäure und 3,52 g p-Methoxybenzylamin in 60 ml abs. THF werden innerhalb 20 Minuten 4,16 g 1-Hydroxybenztriazol und 5,63 g Dicyclohexylcarbodiimid in 60 ml THF zugetropft. Man rührt das Reaktionsgemisch 48 Stunden, filtriert den ausgefallenen Dicyclohexylharnstoff ab, wäscht diesen mehrmals mit THF und dampft das Filtrat ein. Das erhaltene Rohprodukt enthält Dicyclohexylharnstoff und Hydroxybenztriazol als Verunreinigung. Nach Chromatographieren des Gemisches an Silicagel (System: Toluol; Toluol/Essigester-1:4) und Kristallisation der reinen Fraktionen aus Methylenchlorid/Aether wird die Titelverbindung vom F. 122-124° erhalten. [α] = -7 ± 1° (1,112 % in Chloroform).

Beispiel 45: (2S,3R)-2-Brom-3-tert-butyldimethylsilyloxybuttersäure-p-methoxybenzylamid (Ausgangsprodukt)

Eine Lösung von 7,8 g (2S,3R)-2-Brom-3-hydroxybuttersäure-p-methoxybenzylamid in 300 ml Dimethylformamid wird mit 4,875 g tert-Butyldimethylchlorsilan und 3,315 g Imidazol versetzt und 24 Stunden bei Raumtemperatur gerührt. Das Reaktionsgemisch wird darauf im Hochvakuum zu einem Sirup eingeengt und nach Zugabe von eiskalter, gesättigter $NaHCO_3$-Lösung zweimal mit Chloroform ausgeschüttelt. Die organischen Extrakte werden bei 0° nacheinander mit $NaHCO_3$-Lösung, 1 %-iger Citronensäure, $NaHCO_3$-Lösung und Wasser gewaschen, getrocknet und eingedampft. Durch Kristallisation des anfallenden Rohproduktes aus Methylenchlorid/Ether/Perolether erhält man die reine Titelverbindung. F. 112-114°; [α] = -23 ± 1° (1,54 % in Chloroform)

Beispiel 46: (2S,3R)-2-Brom-3-tert-butyldimethylsilyloxybuttersäure-N-tert-butoxycarbonylmethyl-1-N-p-methoxybenzylamid (Ausgangsprodukt)

Zu einem bei 0° unter Argonatmosphäre gerührten Gemisch von 550 mg einer 55-66 %igen Dispersion von Natriumhydrid in Oel und 1,52 g Bromessigsäure-tert-butylester in 25 ml THF wird eine Lösung von 4,17 g (2S,3R)-2-Brom-3-tert-butyldimethylsilyloxybuttersäure-p-methoxybenzylamid in 10 ml THF getropft. Nach 30 Minuten Reaktionszeit lässt man auf Raumtemperatur erwärmen und rührt weitere 90 Minuten. Das Reaktionsgemisch wird von ungelöstem Rückstand abfiltriert, der Filterrückstand mit THF gewaschen und das Filtrat eingedampft. Nach Chromatographieren des Rohproduktes an 400 g Silicagel (System: Toluol; Toluol/Essigester-95:5) erhält man die reine, amorphe Titelverbindung. [α] = +32 ± 1° (0,4 % in Chloroform).

Beispiel 47: 1-p-Methoxybenzyl-3(S)-(1'-(R)-hydroxyethyl)-4(S)-tert-butoxycarbonyl-2-azetidinon

12,45 g Tetrabutylammoniumfluorid-trihydrat werden mit 30 g Molekularsieb (Typ 4171/16) in THF 22 Stunden lang bei Raumtemperatur dehydratisiert. Die Mischung wird auf 0-5° abgekühlt, mit einer Lösung von 3,0 g (2S,3R)-2-Brom-3-tert-butyldimethylsilyloxybuttersäure-N-tert-butoxycarbonylmethyl-N-p-methoxybenzylamid versetzt und 2 Stunden bei Raumtemperatur gerührt. Das Molekularsieb wird abfiltriert, mit 80 ml Methylenchlorid nachgewaschen, das Filtrat mit 500 ml Aether verdünnt und zweimal mit Phosphatpuffer-Lösung (pH 8) gewaschen. Die organische Phasen werden getrocknet und eingedampft. Das anfallende Rohprodukt wird in Methylenchlorid aufgenommen, die Lösung nacheinander mit 1N-Schwefelsäure, Wassser und $NaHCO_3$-Lösung gewaschen, getrocknet und eingedampft. Nach anschliessendem Chromatographieren an Silicagel und Kristallisation der einheitlichen Fraktionen aus Methylenchlorid/Aether/Petrolether wird die reine Titelverbindung vom F. 85-87° und [α] = +13 ± 1° (1,20 % in Chloroform) erhalten.

Beispiel 48: 1-p-Methoxybenzyl-3(S)-(1'-(R)-trichlorethoxycarbonyloxyethyl)-4-(S)-tert-butoxycarbonyl-2-azetidinon

500 mg 1-p-Methoxybenzyl-(3)-S-(1'-(R)-hydroxyethyl)-4-(R)-tert-butoxycarbonyl-2-azetidinon werden in 7,5 ml Methylenchlorid gelöst. Zu der auf 0° abgekühlten Lösung setzt man unter Argonatmosphäre und Rühren 0,27 ml Triethylamin, 0,27 ml Chlorameisensäure-2,2,2,-trichlorethylester und 195 mg Dimethylaminopyridin hinzu, rührt bei 0° 1 Stunde weiter und lässt das Gemisch 18 Stunden bei 5° stehen. Dann giesst man das Gemisch auf Eiswasser, verdünnt dieses mit Methylenchlorid, wäscht die organische Phase nacheinander mit 5 %-iger Citronensäure und $NaHCO_3$-Lösung, trocknet und dampft im Wasserstrahlvakuum ein. Nach Chromatographieren an Silicagel (System: Toluol; Toluol/Essigester 95:5 und 90:10 und Umkristallisieren der einheitlichen Fraktionen aus Methylenchlorid/Aether/Petrolether erhält man die Titelverbindung. F. 102-104°; [α] = +65 ± 1° (1,29 % in Chloroform).

Beispiel 49: 3(S)-(1'(R)-Trichlorethoxycarbonyloxyethyl)-4-(S)-tert-butoxycarbonyl-2-azetidinon

Eine auf -10° abgekühlte Lösung von 510 mg 1-p-Methoxybenzyl-3(S)-(1'-(R)-trichlorethoxycarbonyloxyethyl)-4-(S)-tert-butoxycarbonyl-2-azetidinon in 10 ml Acetonitril wird 15 Minuten gerührt, mit 2,25 g Cer(IV)-ammoniumnitrat in 6,25 ml Wasser versetzt und 30 Minuten weitergerührt. Das Reaktionsgemisch wird auf Eiswasser gegossen und mit Essigester extrahiert. Die organische Phasen wäscht man mit gesättigter, wässriger $NaHCO_3$-Lösung, trocknet und dampft ein. Nach Chromatographieren des Rohproduktes an 50 g Silicagel und anschliessender Kristallisation der reinen Fraktionen aus Methylenchlorid/Aether/Petrolether werden 285 mg reine Titelverbindung erhalten. F. 138-140°; [α] = +29 ± 1° (0,917 % in Chloroform).

Beispiel 50: 3-(S)-(1'-(R)-Trichlorethoxycarbonyloxyethyl)-4(S)-carbonsäure-2-azetidinon

282 mg 3(S)-(1'-(R)-Trichlorethoxycarbonyloxyethyl)-4-(S)-tert-butoxycarbonyl-2-azetidinon werden bei 0° in 10 ml Trifluoressig säure gelöst. Nach einer Stunde Reaktionszeit bei Raumtemperatur wird das Reaktionsgemisch im Hochvakuum eingedampft und die erhältliche Titelverbindung ohne Reinigung weiterverarbeitet; [α]= + 25 ± 1° (0,834 % in Chloroform).

Beispiel 51: (2R,3R)-2,3-Epoxybuttersäure-p-methoxybenzylamid (Ausgangsprodukt)

Eine Lösung von 6,04 g (20 mM) (2R,3R)-2-Brom-3-hydroxybuttersäure-p-methoxybenzylamid in 150 ml Methylenchlorid wird mit 50 ml 50 %iger NaOH-Lösung und 456 mg (2 mM) Benzyltriethylammoniumchlorid versetzt. Das zweiphasige Gemisch wird während 20 Stunden bei Raumtemperatur stark gerührt. Die organische Schicht wird abgetrennt und die wässrige Phase mit Metylenchlorid nachextrahiert. Die vereinigten Methylenchlorid-Lösungen werden getrocknet und eingedampft. Das anfalende Rohprodukt wird an 40-facher Gewichtsmenge Silicagel im System Methylenchlorid/Methanol-99:1 chromatographiert. Nach Kristallisation der reinen Fraktionen aus Methylenchlorid-Aether-Petrolether wird die Titelverbindung, F.: 75-76°, erhalten.

Beispiel 52: (2R,3R)-2,3-Epoxybuttersäure-N-tert-butoxycarbonylmethyl-N-p-methyl-N-p-methoxybenzylamid (Ausgangsprodukt)

Zu einer bei 0° unter Argonatmospähre gerührten Mischung von 550 mg Natriumhydrid-Dispersion (55-60 % in Oel) und 1,52 ml Bromessigsäure-tert-butylester in 25 ml THF wird eine Lösung von 2,21 g (2R,3R)-2,3-Epoxybuttersäure-p-methoxybenzylamid in 100 ml THF zugetropft. Man erwärmt auf Raumtemperatur und rührt das Reaktionsgemisch 1 Stunde weiter (Kontrolle des Reaktionsablaufs mittels Dünnschichtchromatographie). Gesamtreaktionszeit: 90 Minuten. Die unlöslichen Anteile werden abfiltriert und mit THF gewaschen und die vereinigten Filtrate eingedampft. Das anfallende Rohprodukt reinigt man durch Chromatographieren an 150 g Silicagel (System: Toluol, Toluol/Edsigester-80:20. Durch Eindampfen der reinen Fraktionen erhält man die amorphe Titelverbindung. IR-Spektrum: Banden u.a. bei 1740, 1670, 1650, 1615, 1517, 1465, 1360 und 1035.

Beispiel 53: 1-p-Methoxybenzyl-3-(S)-(1'-(R)-hydroxyethyl)-4-(S)-tert-butoxycarbonyl-2-azetidinon

9,23 g Tetrabutylammoniumfluorid-trihydrat werden mit 40 g Molekularsieb (Typ 4171/16 - bei 300° vorgetrocknet) in 80 ml THF 16 Stunden bei 5° stehen gelassen. Die Mischung wird auf 0° abgekühlt, mit einer Lösung von 2,8 g (2R,3R)-2,3-Epoxybuttersäure-N-tert-butoxycarbonylmethyl-N-methoxybenzylamid in 20 ml THF versetzt und 2 Stunden bei 0-5° gerührt. Das Molekularsieb wird unter Nachwaschen mit THF abfiltriert und das Filtrat direkt auf eine in Toluol bereitete Säule mit 250 g Silicagel aufgetragen. Die eingedampften, mit Toluol/Essigester- 70:30-Gemisch eluierten Fraktionen werden in Methylenchlorid aufgenommen, nacheinander zweimal mit 1-N Schwefelsäure, mit gesättigter, wässriger $NaHCO_3$-Lösung und mit Wasser gewasschen, getrocknet und eingedampft. Nach Kurzchromato graphie an Silicagel (Toluol, Toluol/Essigester-60:40) und Kristallisation aus Methylenchlorid/Ether/Petrolether wird die reine Titelverbindung vom F. 85-87° erhalten. (Identisch mit dem nach Angaben im Beispiel 47 erhältlichen Produkt.)

Beispiel 54: 1-p-Methoxybenzyl-3-(S)-(1'-(R)-trichlorethoxycarbonyloxyethyl)-4(S)-carbonsäure-2-azetidinon

350 mg 1-p-Methoxybenzyl-3(S)-(1'-(R)-trichlorethoxycarbonyloxyethyl)-4-(S) -tert-butoxycarbonyl-2-azetidinon werden bei 0° in 10 ml Trifluoressigsäure gelöst. Nach einer Stunde Reaktionszeit bei Raumtemperatur wird das Reaktionsgemisch im Hochvakuum eingedampft und die erhältliche Titelverbindung, die kein Ausgangsmaterial enthält, ohne Reinigung weiterverarbeitet. I.R.-Spektrum: Banden u.a. bei 1768, 1722, 1611, 1584, 1515 und 1035.

Beispiele 55: 1-p-Methoxybenzyl-3-(S)-(1'-(R)-allyloxycarbonyloxyethyl)-4-(S)-tert-butoxycarbonyl-2-azetidinon

Eine Lösung von 2 g 1-p-Methoxybenzyl-3-(S)-(1'-(R)-hydroxyethyl)-4-(S)-tert-butoxycarbonyl-2-azetidinon in 24 ml Methylenchlorid wird bei 0° mit 24 ml 1-N NaOH, 820 mg Tetrabutylammoniumhydrogensulfat und 1 ml Chlorameisensäureallylester versetzt und heftig gerührt. Nach 20 und 40 Minuten Reaktionszeit werden weitere Portionen (je 1 ml) Chlorameisensäureallylester zugegeben. Das Reaktionsgemisch wird mit Methylenchlorid verdünnt, die wässrige Phase abgetrennt und die organische Schicht nacheinander mit 5%-iger wässriger Citronensäure und 8%-iger wässriger $NaHCO_3$-Lösung gewaschen, getrocknet und eingedampft. Nach chromatographischer Reinigung wird die reine, amorphe Titelverbindung. I.R.-Spektrum: Banden u.a. bei 1765, 1745(sh), 1615, 1593, 1515, 1160 und 1035.

Beispiel 56: 1-p-Methoxybenzyl-3-(S)-(1'-(R)-allyloxycarbonyloxyethyl-4(S)-carbonsäure-2-azetidinon

1,6 g 1-p-Methoxybenzyl-3-(S)-(1'-(R)-allyloxycarbonyloxyethyl)-4-(S)-tert-butoxycarbonyl-2-azetidinon werden bei 0° in 10 ml Trifluoressigsäure gelöst. Nach einer Stunde Reaktionszeit bei Raum temperatur wird das Reaktionsgemisch im Hochvakuum eingedampft und die erhältliche Titelverbindung ohne Reinigung weiterverarbeitet. $[\alpha] = +85 \pm 1°$ (1,0 % in Chloroform).

## Ansprüche

1. Verfahren zur Herstellung von (3S)-3,4-trans-disubstituierten Azetidinonen der Formel (I)

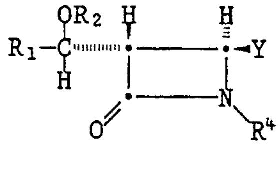

(I),

worin $R_1$ Wasserstoff oder $C_1$-$C_7$-Alkyl,
$R_2$ Wasserstoff oder eine Hydroxyschutzgruppe, Y die Gruppen -S(=O)-$R_3$ oder -S(=O)$_2$-$R_3$, worin $R_3$ einen tertiären $C_4$-$C_7$-Alkyl-, Cycloalkyl-$C_1$-$C_7$-Alkyl-, Aryl-, oder einen durch $C_1$-$C_7$-Alkoxy, $C_1$-$C_7$-Alkyl, Nitro und/oder Halogen substituierten Arylrest wie 4-Methoxyphenyl, 4-Nitrophenyl, 2-, 3-, oder 4-Chlorphenyl, Tolyl, oder einen Triaryl-$C_1$-$C_7$-Alkylrest, welcher jeweils durch das tertiäre C-Atom mit dem Schwefelatom verbunden ist, darstellt, oder die Gruppe -C(=O)O-$R_3'$ , worin $R_3'$ die gleiche Bedeutung besitzt wie $R_3$ und $R_3'$ durch jeweils das tertiäre C-Atom mit dem Sauerstoffatom der Carboxygruppe verbunden ist, oder eine Carboxyschutzgruppe darstellt,
und $R_4$ Wasserstoff oder eine Aminoschutzgruppe $R_4'$ bedeutet, und worin das 4-C-Atom des Azetidinonrings die R-Konfiguration hat, wenn Y die Gruppen -S(=O)-$R_3$ oder -S(=O)$_2$-$R_3$ bedeutet, und die S-Konfiguration hat, wenn Y die Gruppe -C(=O)-O-$R_3'$ bedeutet, und wobei das 1'-C-Atom der Seitenkette die R- oder die S-Konfiguration hat, wenn $R_1$ $C_1$-$C_7$-Alkyl bedeutet, durch Ringschluss einer Verbindung der Formel (II)

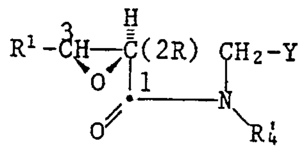

(II)

worin Y und $R_1$ die unter Formel (I) genannten Bedeutungen haben, $R_4'$ eine geeignete Aminoschutzgruppe darstellt und das 2-C-Atom die R- und, wenn $R_1$ $C_1$-$C_7$-Alkyl bedeutet, das 3-C-Atom die R- oder die S-Konfiguration hat, oder einer Verbindung der Formel (III)

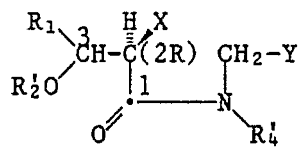

(III)

worin Y und $R_1$ die unter Formel (I) genannten Bedeutungen haben, $R_2'$ Wasserstoff oder eine unter den Bedingungen des Ringschlussverfahrens nicht abspaltbare Hydroxyschutzgruppe, $R_4'$ eine geeignete Aminoschutzgruppe, X eine nukleofuge Abgangsgruppe darstellt und das 2-C-Atom die R- und, wenn $R_1$ $C_1$-$C_7$-Alkyl bedeutet, das 3-C-Atom die R- oder die S-Konfiguration hat, in einem aprotischen, organischen Lösungsmittel unter Ausschluss von Wasser, dadurch gekennzeichnet, dass man den Ringschluss mittels eines Tetra-$C_1$-$C_7$-Alkylammoniumfluorids bei Temperaturen zwischen -10 °C und Raumtemperatur durchführt und, wenn erwünscht, eine erhältliche Verbindung der Formel (I) in eine andere definitionsgemässe Verbindung der Formel (I) umwandelt.

**2.** Verfahren gemäss Anspruch 1 zur Herstellung von (3S)-3,4-trans-disubstituierten Azetidinonen der Formel (I), worin Y eine Gruppe -S(=O)-$R_3$ oder -S(=O)$_2$-$R_3$ bedeutet.

**3.** Verfahren gemäss Anspruch 1 zur Herstellung von (3S)-3,4-trans-disubstituierten Azetidinonen der Formel (I), worin $R_1$ Wasserstoff oder Methyl, $R_2$ Wasserstoff oder eine Hydroxyschutzgruppe, Y die Gruppen -S(=O)$_2$-$R_3$ oder C(=O)-O-$R_3'$ , worin $R_3$ bzw. $R_3'$ tert-$C_4$-$C_7$-Alkyl oder Phenyl bedeuten, und $R_4$ Wasserstoff oder eine Aminoschutzgruppe $R_4'$ bedeutet.

**4.** Verfahren gemäss Anspruch 3 zur Herstellung von (3S)-3,4-trans-disubstituierten Azetidinonen der Formel (I), worin Y die Gruppe -S(=O)$_2$-$R_3$ und $R_3$ tert-$C_4$-$C_7$-Alkyl oder Phenyl bedeuten.

**5.** Verfahren gemäss Anspruch 3 zur Herstellung von (3S)-3,4-trans-disubstituierten Azetidinonen der Formel (I), worin $R_2$ Wasserstoff, 2,2,2-Trichlorethoxycarbonyl, Allyloxycarbonyl, Benzyloxycarbonyl, p-Nitrobenzyloxycarbonyl, Trimethylsilyl oder Dimethyl-tert-butylsilyl $R_4$ Wasserstoff, 4-Methoxybenzyl, 2,4-Dimethoxybenzyl, 4-Methoxyphenyl oder 3,4-Dimethoxyphenyl, bedeuten, und worin y die im Anspruch 3 angegebene Bedeutung hat.

**6.** Verfahren gemäss Anspruch 4 zur Herstellung von (3S)-3,4-trans-disubstituierten Azetidinonen der Formel (I), worin $R_2$ Wasserstoff, 2,2,2-Trichlorethoxycarbonyl, Allyloxycarbonyl, Benzyloxycarbonyl, p-Nitrobenzyloxycarbonyl, Trimethylsilyl oder Dimethyl-tert-butylsilyl $R_4$ Wasserstoff, 4-Methoxybenzyl,

2,4-Dimethoxybenzyl, 4-Methoxyphenyl oder 3,4-Dimethoxyphenyl, bedeuten, und worin y die im Anspruch 4 angegebene Bedeutung hat.

7. Verfahren gemäss Anspruch 1 zur Herstellung von (3S)-3,4-trans-disubstituierten Azetidinonen der Formel (I), dadurch gekennzeichnet, dass man Ausgangsmaterialien der Formel (II) verwendet, worin $R_1$ Wasserstoff oder Methyl, Y die Gruppen $-S(=O)_2-R_3$ oder $-C(=O)-O-R_3'$ , worin $R_3$ bzw. $R_3'$ tert-Butyl oder Phenyl bedeuten, und $R_4'$ 4-Methoxybenzyl, 2,4-Dimethoxybenzyl, 4-Methoxyphenyl oder 3,4-Dimethoxyphenyl bedeutet.

8. Verfahren gemäss Anspruch 7 zur Herstellung von (3S)-3,4-trans-disubstituierten Azetidinonen der Formel (I), dadurch gekennzeichnet, dass man Ausgangsmaterialien der Formel (II) verwendet, worin Y die Gruppe $-S(=O)_2-R_3$ bedeutet.

9. Verfahren nach Anspruch 1, dadurch gekennzeichnet, dass man in Gegenwart von Tetra-n-butylammoniumfluorid zyklisiert.

10. Verfahren nach Anspruch 1, dadurch gekennzeichnet, dass man die Reaktion in Tetrahydrofuran als Lösungsmittel durchführt.

11. Verfahren nach Anspruch 1, dadurch gekennzeichnet, dass man eine Aminoschutzgruppe $R_4'$ mit der Bedeutung p-Methoxybenzyl mit Cer(IV)-ammoniumnitrat abspaltet.

12. Verfahren gemäss einem der Ansprüche 1-10, dadurch gekennzeichnet, dass man 1-(2,4-Dimethoxybenzyl)-3-(S)-Hydroxymethyl-4-(R)-tert-butylsulphonyl-2-azetidinon herstellt.

13. Verfahren gemäss einem der Ansprüche 1-11, dadurch gekennzeichnet, dass man 3-(S)-Hydroxymethyl-4-(R)-tert-butylsulfonyl-2-azetidinon herstellt.

14. Verfahren gemäss einem der Ansprüche 1-10, dadurch gekennzeichnet, dass man 1-p-Methoxybenzyl-3-(S)-hydroxymethyl-4-(R)-tert-butylsulfonyl-2-azetidinon herstellt.

15. Verfahren gemäss einem der Ansprüche 1-10, dadurch gekennzeichnet, dass man 1-p-Methoxybenzyl-3-(S)-(1'-(R)-hydroxyethyl)-4-(R)-tert-butylsulfonyl-2-azetidinon herstellt.

16. Verfahren gemäss einem der Ansprüche 1-11, dadurch gekennzeichnet, dass man 3-(S)-(1'-(R)-hydroxyethyl)-4-R-tert-butylsulfonyl-2-azetidinon herstellt.

## Claims

1. Process for the manufacture of (3S )-3,4-trans-disubstituted azetidinones of the formula (I)

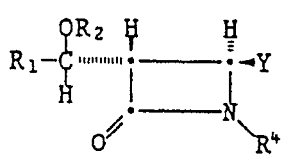

(I)

in which
$R_1$ represents hydrogen or $C_1$-$C_7$alkyl,
$R_2$ represents hydrogen or a hydroxy-protecting group,
Y represents the group $-S(=O)-R_3$ or $-S(=O)_2-R_3$ wherein $R_3$ represents a tertiary $C_4$-$C_7$alkyl, cycloalkyl-$C_1$-$C_7$alkyl, aryl or $C_1$-$C_7$alkoxy-, $C_1$-$C_7$alkyl-, nitro- and/or halo-substituted aryl radical, such as 4-methoxyphenyl, 4-nitrophenyl, 2-, 3- or 4-chlorophenyl, tolyl, or a triaryl-$C_1$-$C_7$alkyl radical, which is in each case bonded to the sulphur atom by the tertiary carbon atom, or the group $-C(=O)-O-R_3'$ wherein $R_3'$ has the same meaning as $R_3$ and $R_3'$ is bonded to the oxygen atom of the carboxy group in each case by the tertiary carbon atom, or is a carboxy-protecting group, and
$R_4$ represents hydrogen or an amino-protecting group $R_4'$, and in which the 4-carbon atom of the

azetidinone ring has the R -configuration if Y represents the group -S(=O)-$R_3$ or -S(=O)$_2$-$R_3$, and the S -configuration if Y represents the group -C(=O)-O-$R_3$', and wherein the 1'-carbon atom of the side chain has the R - or S -configuration if $R_1$ represents $C_1$-$C_7$alkyl, by cyclisation of a compound of the formula (II)

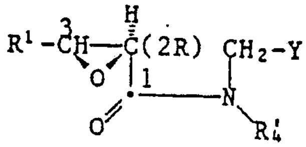

(II)

in which Y and $R_1$ have the meanings given under formula (I), $R_4$' represents a suitable amino-protecting group, and the 2-carbon atom has the R -configuration and, if $R_1$ represents $C_1$-$C_7$alkyl, the 3-carbon atom has the R - or the S -configuration, or a compound of the formula (III)

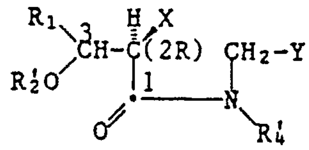

(III)

in which Y and $R_1$ have the meanings given under formula (I), $R_2$' represents hydrogen or a hydroxy-protecting group that cannot be removed under the conditions of the cyclisation operation, $R_4$' represents a suitable amino-protecting group, X represents a nucleofugal leaving group, and the 2-carbon atom has the R -configuration and, if $R_1$ represents $C_1$-$C_7$alkyl, the 3-carbon atom has the R -or the S -configuration, in an aprotic organic solvent in the absence of water, characterised in that the cyclisation is carried out by means of a tetra-$C_1$-$C_7$alkylammonium fluoride at temperatures of between -10 °C and room temperature, and, if desired, a resulting compound of the formula (I) is converted into a different compound of the formula (I) in accordance with the definition.

2. Process according to claim 1 for the manufacture of (3S )-3,4-trans -disubstituted azetidinones of the formula (I) in which Y represents a group -S(=O)-$R_3$ or -S(=O)$_2$-$R_3$.

3. Process according to claim 1 for the manufacture of (3S )-3,4-trans -disubstituted azetidinones of the formula (I) in which $R_1$ represents hydrogen or methyl, $R_2$ represents hydrogen or a hydroxy-protecting group, Y represents the group -S(=O)$_2$-$R_3$ or -C(=O)-O-$R_3$' wherein $R_3$ or $R_3$' represents tert.-$C_4$-$C_7$alkyl or phenyl, and $R_4$ represents hydrogen or an amino-protecting group $R_4$'.

4. Process according to claim 3 for the manufacture of (3S )-3,4-trans -disubstituted azetidinones of the formula (I) in which Y represents the group -S(=O)$_2$-$R_3$ and $R_3$ represents tert.-$C_4$-$C_7$alkyl or phenyl.

5. Process according to claim 3 for the manufacture of (3S )-3,4-trans -disubstituted azetidinones of the formula (I) in which $R_2$ represents hydrogen, 2,2,2-trichloroethoxycarbonyl, allyloxycarbonyl, benzyloxycarbonyl, p -nitrobenzyloxycarbonyl, trimethylsilyl or dimethyl-tert.-butylsilyl, $R_4$ represents hydrogen, 4-methoxybenzyl, 2,4-dimethoxybenzyl, 4-methoxyphenyl or 3,4-dimethoxyphenyl, and in which Y has the meaning given in claim 3.

6. Process according to claim 4 for the manufacture of (3S )-3,4-trans -disubstituted azetidinones of the formula (I) in which $R_2$ represents hydrogen, 2,2,2-trichloroethoxycarbonyl, allyloxycarbonyl, benzyloxycarbonyl, p-nitrobenzyloxycarbonyl, trimethylsilyl or dimethyl-tert.-butylsilyl, $R_4$ represents hydrogen, 4-methoxybenzyl, 2,4-dimethoxybenzyl, 4-methoxyphenyl or 3,4-dimethoxyphenyl, and in which Y has the meaning given in claim 4.

7. Process according to claim 1 for the manufacture of (3S )-3,4-trans -disubstituted azetidinones of the formula (I), characterised in that there are used starting materials of the formula (II) in which $R_1$ represents hydrogen or methyl, Y represents the group -S(=O)$_2$-$R_3$ or -C(=O)-O-$R_3$' wherein $R_3$ or $R_3$' represents tert.-butyl or phenyl, and $R_4$' represents 4-methoxybenzyl, 2,4-dimethoxybenzyl, 4-methoxyphenyl or 3,4-dimethoxyphenyl.

8. Process according to claim 7 for the manufacture of (3S)-3,4-trans-disubstituted azetidinones of the formula (I), characterised in that there are used starting materials of the formula (II) in which Y represents the group $-S(=O)_2-R_3$.

9. Process according to claim 1, characterised in that cyclisation is carried out in the presence of tetra-n-butylammonium fluoride.

10. Process according to claim 1, characterised in that the reaction is carried out in tetrahydrofuran as solvent.

11. Process according to claim 1, characterised in that an amino-protecting group $R_4'$ that represents p-methoxybenzyl is removed with cerium(IV)-ammonium nitrate.

12. Process according to any one of claims 1 to 10, characterised in that 1-(2,4-dimethoxybenzyl)-3-(S)-hydroxymethyl-4-(R)-tert.-butylsulphonyl-2-azetidinone is manufactured.

13. Process according to any one of claims 1 to 11, characterised in that 3-(S)-hydroxymethyl-4-(R)-tert.-butylsulphonyl-2-azetidinone is manufactured.

14. Process according to any one of claims 1 to 10, characterised in that 1-p-methoxybenzyl-3-(S)-hydroxymethyl-4-(R)-tert.-butylsulphonyl-2-azetidinone is manufactured.

15. Process according to any one of claims 1 to 10, characterised in that 1-p-methoxybenzyl-3-(S)-(1'-(R)-hydroxyethyl)-4-(R)-tert.-butylsulphonyl-2-azetidinone is manufactured.

16. Process according to any one of claims 1 to 11, characterised in that 3-(S)-(1'-(R)-hydroxyethyl)-4-R-tert.-butylsulphonyl-2-azetidinone is manufactured.

## Revendications

1. Procédé de préparation d'azétidinones (3S)-3,4-trans-disubstituées de formule

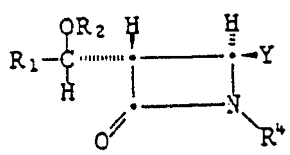

(I)

dans laquelle $R_1$ représente l'hydrogène ou un alkyle $C_1$-$C_7$, $R_2$ l'hydrogène ou un groupe protecteur d'hydroxy, Y les groupes $-S(=O)-R_3$ ou $-S(=O)_2-R_3$ dans lesquels $R_3$ représente un alkyle $C_4$-$C_7$ tertiaire, un cycloalkyle (alkyle $C_1$-$C_7$), un aryle ou un reste aryle substitué par un alcoxy $C_1$-$C_7$, un alkyle $C_1$-$C_7$, un nitro et/ou un halogène tel que le 4-méthoxyphényle, le 4-nitrophényle, 2-, 3- ou 4-chloro-phényle, tolyle ou un reste triaryl-(alkyle $C_1$-$C_7$) qui, dans chaque cas est relié par l'atome de C tertiaire à l'atome de soufre, ou le groupe $-C(=O)-O-R_3'$, dans lequel $R_3'$ a la même signification que $R_3$, et $R_3'$ est relié dans chaque cas par l'atome de C tertiaire à l'atome d'oxygène du groupe carboxy, ou un groupe protecteur de carboxy, et $R_4$ représente l'hydrogène ou un groupe protecteur d'amino $R_4'$, et dans laquelle l'atome 4 de C du cycle azétidinone a la configuration R, quand Y représente les groupes $-S(=O)-R_3$ ou $-S(=O)_2-R_3$, et a la configuration S, quand Y représente le groupe $-C(=O)-O-R_3'$, et dans laquelle l'atome 1' de C de la chaîne latérale a la configuration R ou S, quand $R_1$ représente un alkyle $C_1$-$C_7$, par cyclisation d'un composé de formule (II)

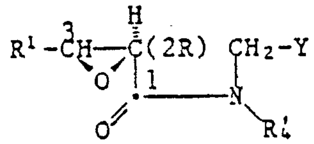

(II)

dans laquelle Y et $R_1$ ont les significations données pour la formule (I), $R'_4$ représente un groupe protecteur d'amino approprié et le carbone 2 de C a la configuration R et, quand $R_1$ représente un alkyle $C_1$-$C_7$, l'atome 3 de C a la configuration R ou S, ou d'un composé de formule (III)

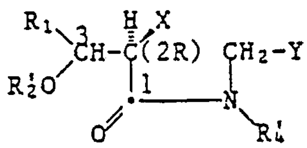

(III)

dans laquelle Y et $R_1$ ont les significations données pour la formule (I), $R'_2$ représente l'hydrogène ou un groupe protecteur d'hydroxy non éliminable dans les conditions de cyclisation, $R'_4$ est un groupe protecteur d'amino approprié, X est un groupe labile nucléofuge et l'atome 2 de C a la configuration R et, quand $R_1$ représente un alkyle $C_1$-$C_7$, l'atome 3 de C a la configuration R ou S, dans un solvant organique aprotique anhydre, caractérisé en ce qu 'on réalise la cyclisation au moyen d'un fluorure de tétra-(alkyle $C_1$-$C_7$)-ammonium à des températures comprises entre -10 °C et la température ambiante et, si c'est souhaité, on transforme un composé obtenu de formule (I) en un autre composé selon la définition de la formule (I).

2. Procédé selon la revendication 1 de préparation d'azétidinones (3S)-3,4-trans-disubstituées de formule (I) dans laquelle Y représente un groupe $-S(=O)-R_3$ ou $-S(=O)_2-R_3$.

3. Procédé selon la revendication 1 de préparation d'azétidinones (3S)-3,4-trans-disubstituées de formule (I), dans laquelle $R_1$ représente l'hydrogène ou le méthyle, $R_2$ l'hydrogène ou un groupe protecteur d'hydroxy, Y les groupes $-S(=O)_2-R_3$ ou $-C(=O)-O-R_3$ dans lesquels $R_3$ ou $R'_3$ représentent un alkyle $C_4$-$C_7$ tertiaire ou un phényle, et $R_4$ l'hydrogène ou un groupe protecteur d'amino $R'_4$ .

4. Procédé selon la revendication 3 de préparation d'azétidinones (3S)-3,4-trans-disubstituées de formule (I) dans laquelle Y est le groupe $-S(=O)_2-R_3$ et $R_3$ un alkyle $C_4$-$C_7$ tertiaire ou un phényle.

5. Procédé selon la revendication 3 de préparation d'azétidinones (3S)-3,4-trans-disubstituées de formule (I) dans laquelle $R_2$ est l'hydrogène, le chloroéthoxycarbonyle, allyloxycarbonyle, benzyloxycarbonyle, p-nitrobenzyloxycarbonyle, triméthylsilyle ou diméthyl-tert-butylsilyle, $R_4$ l'hydrogène, le 4-méthoxybenzyle, 2,4-diméthoxybenzyle, 4-méthoxyphényle ou 3,4-diméthoxyphényle, et dans laquelle Y a la signification donnée dans la revendication 3.

6. Procédé selon la revendication 4 de préparation d'azétidinones (3S)-3,4-trans-disubstituées de formule (I) dans laquelle $R_2$ représente l'hydrogène, le 2,2,2-trichloroéthoxycarbonyle, allyloxycarbonyle, benzyloxycarbonyle, p-nitrobenzyloxycarbonyle, triméthylsilyle ou diméthyl-tert-butylsilyle, $R_4$ l'hydrogène, le 4-méthoxybenzyle, 2,4-diméthoxybenzyle, 4-méthoxyphényle ou 3,4-diméthoxyphényle, et dans laquelle Y a la signification donnée dans la revendication 4.

7. Procédé selon la revendication 1 de préparation d'azétidinones (3S)-3,4-trans-disubstituées de formule (I), caractérisé en ce qu'on utilise des matériaux de départ de formule (II) dans lesquels représentent $R_1$ l'hydrogène ou le méthyle, Y les groupes $-S(=O)_2-R_3$ ou $-C(=O)-O-R'_3$ , dans lesquels $R_3$ ou $R'_3$ représentent le tert-butyle ou le phényle, et $R'_4$ représente le 4-méthoxybenzyle, 2,4-diméthoxybenzyle, 4-méthoxyphényle ou 3,4-diméthoxyphényle.

8. Procédé selon la revendication 7 de préparation d'azétidinones (3S)-3,4-trans-disubstituées de formule

(I), caractérisé en ce qu'on utilise des maté riaux de départ de formule (II), dans laquelle Y représente le groupe $-S(=O)_2-R_3$.

**9.** Procédé selon la revendication 1, caractérisé en ce qu'on cyclise en présence de fluorure de tétra-n-butylammonium.

**10.** Procédé selon la revendication 1, caractérisé en ce qu'on réalise la réaction dans le tétrahydrofuranne comme solvant.

**11.** Procédé selon la revendication 1, caractérisé en ce qu'on élimine un groupe $R_4'$ protecteur d'amino représenté par le p-méthoxybenzyle avec du nitrate de cérium(IV) ammonique.

**12.** Procédé selon l'une des revendications 1-10, caractérisé en ce qu'on prépare la 1-(2,4-diméthoxybenzyl)-3-(S)-hydroxyméthyl-4-(R)-tert-butylsulfonyl-2-azétidinone.

**13.** Procédé selon l'une des revendications 1-11 caractérisé en ce qu'on prépare la 3-(S)-hydroxyméthyl-4-(R)-tert-butylsulfonyl-2-azétidinone.

**14.** Procédé selon l'une des revendications 1-10, caractérisé en ce qu'on prépare la 1-p-méthoxybenzyl-3-(S)-hydroxyméthyl-4-(R)-tert-butylsulfonyl-2-azétidinone.

**15.** Procédé selon l'une des revendications 1-10 caractérisé en ce qu'on prépare la 1-p-méthoxybenzyl-3-(S)-(1'-(R)-hydroxyéthyl)-4-(R)-tert-butylsulfonyl-2-azétidinone.

**16.** Procédé selon l'une des revendications 1-11 caractérisé en ce qu'on prépare la 3-(S)-(1'-(R)-hydroxyéthyl)-4-R-tert-butylsulfonyl-2-azétidinone.